# EUROPEAN PATENT SPECIFICATION

(11) **EP 1 523 572 B1**
(45) Date of publication and mention of the grant of the patent: **07.05.2008**
(21) Application number: 03762684.3
(22) Date of filing: 09.07.2003
(51) Int. Cl.: C12Q 1/48, C07K 14/245, C12N 15/31, C12N 15/01, C12N 15/63, C12Q 1/02, C07K 14/195, C07K 16/12

(54) **PATHOGENICITY DETERMINANTS WHICH CAN BE USED AS TARGETS FOR DEVELOPING MEANS FOR PREVENTING AND CONTROLLING BACTERIAL INFECTIONS AND/OR SYSTEMIC DISSEMINATION**
PATHOGENE DETERMINANTEN ALS ZIELMOLEKÜLE ZUR ENTWICKLUNG VON MITTELN WELCHE BAKTERIELLE INFEKTIONEN UND/ODER SYSTEMISCHE VERBREITUNG VERHINDERN UND KONTROLLIEREN KÖNNEN
DETERMINANTS A PATHOGENICITE POUVANT ETRE UTILISES COMME CIBLES EN VUE DU DEVELOPPEMENT DE MOYENS DE PREVENTION ET DE CONTROLE D'INFECTIONS BACTERIENNES ET/OU DE DISSEMINATION SYSTEMIQUE

(30) Priority: 09.07.2002 FR 0208636
(43) Date of publication of application: 20.04.2005
(73) Proprietor: Mutabilis, 75730 Paris Cedex 15 (FR)
(72) Inventor: ESCAICH, Sonia, F-75012 PARIS (FR)
(74) Representative: Peaucelle, Chantal
(86) International application number: PCT/EP2003/008209
(87) International publication number: WO 2004/005535

(56) References cited:
- WO-A-00/28038
- WO-A-00/44906
- WO-A-99/66049
- US-A- 6 020 121
- YETHON J A ET AL: "Involvement of waaY, waaQ and waaP in the modification of E. coli lipopolysaccharide and their role in the formation of a stable outer membrane" JOURNAL OF BIOLOGICAL CHEMISTRY, AMERICAN SOCIETY OF BIOLOGICAL CHEMISTS, BALTIMORE, MD, US, vol. 273, no. 41, 9 October 1998 (1998-10-09), pages 26310-26316, XP002118875 ISSN: 0021-9258
- HEINRICHS DAVID E ET AL: "Molecular basis for structural diversity in the core regions of the lipopolysaccharides of Escherichia coli and Salmonella enterica." MOLECULAR MICROBIOLOGY, vol. 30, no. 2, October 1998 (1998-10), pages 221-232, XP002118873 ISSN: 0950-382X
- BURNS STACY M ET AL: "Comparison of loss of serum resistance by defined lipopolysaccharide mutants and an acapsular mutant of uropathogenic Escherichia coli O75:K5." INFECTION AND IMMUNITY, vol. 66, no. 9, 1998, pages 4244-4253, XP002250632 ISSN: 0019-9567 cited in the application
- RUSSO THOMAS A ET AL: "Loss of the O4 antigen moiety from the lipopolysaccharide of an extraintestinal isolate of Escherichia coli has only minor effects on serum sensitivity and virulence in vivo." INFECTION AND IMMUNITY, vol. 63, no. 4, 1995, pages 1263-1269, XP002250633 ISSN: 0019-9567 cited in the application
- HONG MEI ET AL: "Effect of mutations in Shigella flexneri chromosomal and plasmid-encoded lipopolysaccharide genes on invasion and serum resistance." MOLECULAR MICROBIOLOGY, vol. 24, no. 4, 1997, pages 779-791, XP008020612 ISSN: 0950-382X
- MERINO SUSANA ET AL: "Cloning and sequencing of the Klebsiella pneumoniae O5 wb gene cluster and its role in pathogenesis." INFECTION AND IMMUNITY, vol. 68, no. 5, May 2000 (2000-05), pages 2435-2440, XP002250634 ISSN: 0019-9567
- RAUTEMAA RIINA ET AL: "Complement-resistance mechanisms of bacteria." MICROBES AND INFECTION, vol. 1, no. 10, August 1999 (1999-08), pages 785-794, XP002250635 ISSN: 1286-4579 cited in the application
- SHEA JACQUELINE E ET AL: "Signature-tagged mutagenesis in the identification of virulence genes in pathogens." CURRENT OPINION IN MICROBIOLOGY, vol. 3, no. 5, October 2000 (2000-10), pages 451-458, XP002250636 ISSN: 1369-5274
- DATABASE UNIPROT 1 May 1999 (1999-05-01), "WaaC" XP002284637 Database accession no. Q9ZITI1
- DATABASE UNIPROT 1 May 2000 (2000-05-01), "WaaQ" XP002284638 Database accession no. Q9R9D5
- DATABASE UNIPROT 1 May 2000 (2000-05-01), "WaaP" XP002284639 Database accession no. Q9R9D6
- DATABASE UNIPROT 1 May 2000 (2000-05-01), "WaaG" XP002284640 Database accession no. Q9R2L8
- DATABASE UNIPROT 1 October 1994 (1994-10-01), "RFAF" XP002284641 Database accession no. P37692
- DATABASE UNIPROT 1 May 1999 (1999-05-01), "WaaO" XP002284642 Database accession no. Q9ZIS5
- DATABASE UNIPROT 1 May 1992 (1992-05-01), "GalU" XP002284643 Database accession no. P25520
- DATABASE UNIPROT 1 June 1994 (1994-06-01), "PGMU" XP002284644 Database accession no. P36938
- DATABASE UNIPROT 1 February 1995 (1995-02-01), "KpsD" XP002284645 Database accession no. Q03961
- DATABASE UNIPROT 1 November 1996 (1996-11-01), "Neud" XP002284646 Database accession no. Q46674
- DATABASE UNIPROT 1 July 1993 (1993-07-01), "GmhB" XP002284647 Database accession no. P31546
- DATABASE UNIPROT 1 November 1990 (1990-11-01), "RFAD" XP002284648 Database accession no. P17963
- DATABASE UNIPROT 15 July 1999 (1999-07-15), "rfaE" XP002284649 Database accession no. P76658
- DATABASE UNIPROT 1 March 2002 (2002-03-01), "DltD" XP002284650 Database accession no. Q8VM64
- DATABASE GENESEQ 2 July 2002 (2002-07-02), "DLTA" XP002284651 Database accession no. ABP30484
- KNEIDINGER BERND ET AL: "Biosynthesis pathway of ADP-L-glycero-beta-D-manno-heptose in Escherichia coli" JOURNAL OF BACTERIOLOGY, vol. 184, no. 2, January 2002 (2002-01), pages 363-369, XP002284579 ISSN: 0021-9193
- KNEIDINGER BERND ET AL: "Biosynthesis of nucleotide-activated D-glycero-D-manno-heptose" JOURNAL OF BIOLOGICAL CHEMISTRY, vol. 276, no. 24, 15 June 2001 (2001-06-15), pages 20935-20944, XP002284580 ISSN: 0021-9258
- SISTI FEDERICO ET AL: "In vitro and in vivo characterization of a Bordetella bronchiseptica mutant strain with a deep rough lipopolysaccharide structure" INFECTION AND IMMUNITY, vol. 70, no. 4, April 2002 (2002-04), pages 1791-1798, XP002284581 ISSN: 0019-9567
- YETHON JEREMY A ET AL: "Purification and characterization of Waap from Escherichia coli, a lipopolysaccharide kinase essential for outer membrane stability" JOURNAL OF BIOLOGICAL CHEMISTRY, vol. 276, no. 8, 23 February 2001 (2001-02-23), pages 5498-5504, XP002284582 ISSN: 0021-9258
- YETHON JEREMY A ET AL: "Salmonella enterica serovar typhimurium waaP mutants show increased susceptibility to polymyxin and loss of virulence in vivo" INFECTION AND IMMUNITY, vol. 68, no. 8, August 2000 (2000-08), pages 4485-4491, XP002284583 ISSN: 0019-9567
- GENEVAUX PIERRE ET AL: "Identification of Tn10 insertions in the rfaG, rfaP, and galU genes involved in lipopolysaccharide core biosynthesis that affect Escherichia coli adhesion" ARCHIVES OF MICROBIOLOGY, vol. 172, no. 1, July 1999 (1999-07), pages 1-8, XP002284584 ISSN: 0302-8933
- NGELEKA MUSANGU ET AL: "Characterization of a polysaccharide capsular antigen of septicemic Escherichia coli O115: K "V165": F165 and evaluation of its role in pathogenicity" INFECTION AND IMMUNITY, vol. 60, no. 12, 1992, pages 5048-5056, XP002284585 ISSN: 0019-9567
- DAINES DAYLE A ET AL: "NeuD plays a role in the synthesis of sialic acid in Escherichia coli K1" FEMS MICROBIOLOGY LETTERS, vol. 189, no. 2, 15 August 2000 (2000-08-15), pages 281-284, XP002284613 ISSN: 0378-1097
- YETHON JEREMY A ET AL: "Mutation of the lipopolysaccharide core glycosyltransferase encoded by waaG destabilizes the outer membrane of Escherichia coli by interfering with core phosphorylation" JOURNAL OF BACTERIOLOGY, vol. 182, no. 19, October 2000 (2000-10), pages 5620-5623, XP002284615 ISSN: 0021-9193
- NESPER JUTTA ET AL: "Characterization of Vibrio cholerae O1 El Tor galU and galE mutants: Influence on lipopolysaccharide structure, colonization, and biofilm formation" INFECTION AND IMMUNITY, vol. 69, no. 1, January 2001 (2001-01), pages 435-445, XP002284616 ISSN: 0019-9567
- ABACHIN ERIC ET AL: "Formation of D-alanyl-lipoteichoic acid is required for adhesion and virulence of Listeria monocytogenes" MOLECULAR MICROBIOLOGY, vol. 43, no. 1, January 2002 (2002-01), pages 1-14, XP002284617 ISSN: 0950-382X
- MARTINDALE J ET AL: "Genetic analysisof E. Coli K1 gastrointestinal colonization" MOLECULAR MICROBIOLOGY, vol. 37, no. 6, September 2000 (2000-09), pages 1293-1305, XP002284618 ISSN: 0950-382X
- CHANG HWAN-YOU ET AL: "Virulence and outer membrane properties of a galU mutant of Klebsiella pneumoniae CG43" MICROBIAL PATHOGENESIS, vol. 20, no. 5, 1996, pages 255-261, XP002284619 ISSN: 0882-4010
- REGNI CATHERINE ET AL: "Crystal structure of PMM/PGM: An enzyme in the biosynthetic pathway of P. aeruginosa virulence factors" STRUCTURE (CAMBRIDGE), vol. 10, no. 2, February 2002 (2002-02), pages 269-279, XP002284620 ISSN: 0969-2126
- TANG C ET AL: "PATHOGEN VIRULENCE GENES - IMPLICATIONS FOR VACCINES AND DRUG THERAPY" BRITISH MEDICAL BULLETIN, CHURCHILL LIVINGSTONE, LONDON, GB, vol. 55, no. 2, 1999, pages 387-400, XP008008098 ISSN: 0007-1420
- MOXON R ET AL: "CHALLENGE OF INVESTIGATING BILOGICALLY RELEVANT FUNCTIONS OF VIRULENCE FACTORS IN BACTERIAL PATHOGENS" PHILOSOPHICAL TRANSACTIONS. ROYAL SOCIETY OF LONDON. BIOLOGICAL SCIENCES, ROYAL SOCIETY, LONDON, GB, vol. 355, no. 1397, 25 May 2000 (2000-05-25), pages 643-656, XP001007306 ISSN: 0962-8436
- VALVANO M A ET AL: "The rfaE gene from Escherichia coli encodes a bifunctional protein involved in biosynthesis of the lipopolysaccharide core precursor ADP-L-glycero-D-manno-heptose" JOURNAL OF BACTERIOLOGY, WASHINGTON, DC, US, vol. 182, no. 2, January 2000 (2000-01), pages 488-497, XP000926030 ISSN: 0021-9193
- LEE N G ET AL: "Molecular cloning and characterization of the nontypable Haemophilus influenzae-2019 rfaE gene required for lipopolysaccharide biosynthesis" INFECTION AND IMMUNITY, AMERICAN SOCIETY FOR MICROBIOLOGY. WASHINGTON, US, vol. 63, no. 3, 1995, pages 818-824, XP000953326 ISSN: 0019-9567
- CIESLEWICZ M ET AL: "THERMOREGULATION OF KPSF, THE FIRST REGION 1 GENE IN THE KPS LOCUS FOR POLYSIALIC ACID BIOSYNTHESIS IN ESCHERICHIA COLI K1" JOURNAL OF BACTERIOLOGY, WASHINGTON, DC, US, vol. 178, no. 11, June 1996 (1996-06), pages 3212-3220, XP000877094 ISSN: 0021-9193
- BOYD E F & HARTL D L: "Chromosomal regions specific to pathogenic isolates of Escherichia coli have a phylogenetically clustered distribution" JOURNAL OF BACTERIOLOGY, WASHINGTON, DC, US, vol. 180, no. 5, March 1998 (1998-03), pages 1159-1165, XP002133065 ISSN: 0021-9193
- AUSTIN A E ET AL: "Genetic analysis of lipopolysaccharide core biosynthesis by Escherichia coli k12 insertion mutagenesis of the RFA locus" JOURNAL OF BACTERIOLOGY, WASHINGTON, DC, US, vol. 172, no. 9, September 1990 (1990-09), pages 5312-5325, XP000926028 ISSN: 0021-9193
- STOJILJKOVIC I ET AL: "CLONING AND CHARACTERIZATION OF THE NEISSERIA MENINGITIDIS RFAC GENE ENCODING ALPHA-1,5 HEPTOSYLTRANSFERASE I" FEMS MICROBIOLOGY LETTERS, AMSTERDAM, NL, vol. 151, no. 1, 1997, pages 41-49, XP001007307 ISSN: 0378-1097
- JOHNSON AND RUSSO: "Extraintestinal pathogenic Escherichia coli: "The other bad E. coli"" J LAB CLIN MED, vol. 139, no. 3, March 2002 (2002-03), pages 155-162,
- RUSSO T. A. AND JOHNSON J. R.: "Proposal for a new inclusive designation for extraintestinal pathogenic isolates of Escherichia coli: ExPEC" THE JOURNAL OF INFECTIOUS DISEASES, vol. 181, May 2000 (2000-05), pages 1753-1754, US

## Description

The invention relates to pathogenicity determinants which can be used as targets for developing means for preventing and controlling bacterial infections and/or systemic dissemination.

Current treatments for infectious diseases of bacterial origin are based on the inhibition of essential bacterial targets in vitro using antibiotics. These targets are conserved in many bacterial species and make it possible to treat various types of infection. However, broad-spectrum antibiotics are active on the host's commensal flora, which promotes the acquisition and transfer of mechanisms of resistance to these antibiotics, hence a limiting of the effectiveness of current treatments with antibiotics. A need therefore exists for novel antibacterial treatments.

In this regard, the invention provides a novel strategy, the aim of which is to specifically target pathogenic bacteria without significantly altering their growth at their portal of entry into the host organism, where they are in a situation of commensalism. These pathogens are in particular the bacteria responsible for serious systemic infections, such as *E.coli,* in general *Enterobacteria, Pseudomonas, Acinetobacter, Moraxella* and *Neisseria* and, for the gram positives, the bacteria of the genus *Staphylococcus, Enterococcus* and *Streptococcus.*

It is known, specifically, that the bacteria responsible for serious infections are capable of growth in the presence of serum and are resistant to the bactericidal action of complement. This resistance allows dissemination of the infection, via the blood, to the various tissues of the host's body.

The ability of bacteria to grow in human serum is due to different pathogenicity/virulence factors. Among those frequently cited, mention will be made of the physical barrier, represented by the capsule, for access of complement to the bacterial membrane, the sialic acids of the capsule or of the O antigen which promote binding of factor H to c3b, and particular surface proteins such as PorA *(Neisseria gonorrhoeae),* YadA *(Yersinia pestis)* or protein M *(Streptococcus pyogenes),* which bind factor H, all these factors preventing complement activation.

Other proteins expressed or bound by the pathogens have proved to be important for resistance to complement and cause cleavage of complement factors or inhibit their binding to the surface of the bacterium (Rautemaa R.; Meri S., Microbes and Infection 1999, 1:785:794).

The lipopolysaccharide (LPS) of gram-negative bacteria is known to be a virulence factor, but the role of its various constituents on the resistance to serum has not been established for all bacterial species. For example, in some studies in *E.coli,* the O antigen is considered to be determinant (Burns S.M. Hull S.I. Infect Immun, 1998, Sept 66 (9) :4244-53) ; in other studies, the O antigen is thought to be less determinant than the capsular antigens for resistance to serum (Russo T. et al., Infect Immun, 1995, Apr. 63(4):1263-9). Furthermore, the importance of these factors on intestinal colonization is unknown.

The inventors have carried out a systematic analysis of mutants for inactivation of the genes required for surface polysaccharide synthesis, and have demonstrated, in *Escherichia coli* strains responsible for extra-intestinal infections, EXPEC, which genes are essential for the resistance to serum and the dissemination in the blood. These results are based on the study of the effect of mutations on virulence and intestinal colonization in an animal model.

The invention is therefore directed towards a novel methodology for defining the targets required for virulence, and not essential *in vitro,* and thus providing novel anti-infectious agents specific for pathogenic bacteria, in particular for extra-intestinal *E.coli*, responsible for severe infections, as well as Gram positive strains, such as *Streptococcus agalactiae.* It is also directed towards the products of the genes required for resistance in the serum and virulence *in vivo.*

The method of the invention for identifying and selecting virulence gene targets in pathogenic bacteria is characterized in that it comprises:
- using a strain of the pathogenic microorganism,
- generating mutants for inactivation in the genes encoding the virulence factors,
- determining the virulence of these mutants on an experimental model of infection and their effect on enteric colonization in an axenic mouse model, and
- identifying and selecting the bacteria having a mutation in genes that prove to have all the following features: genes non essential for proliferation *in vitro,* non essential for the commensal colonization, but essential for resistance to serum *in vitro*, and essential, in the host, for dissemination in blood.

The invention also relates to a method such as above defined, comprising the step of assessing the viability of the mutant bacteria *in vitro* and its ability to resist and proliferate in presence of complemented serum.

The pathogenic microorganism is in particular an EXPEC strain of *E.coli.*

The invention also relates to an *in vitro* process for inhibiting the virulence of pathogenic bacteria, comprising targeting with an inhibitor a nucleic acid of SEQ ID N°16, 20, 27 or 28 or a peptide of SEQ ID N°1, 5, 12 or 13, to increase the serum sensibility of the bacteria and attenuate their virulence.

The invention is also directed towards an *in vitro* such as above defined wherein the nucleic acids, expression products thereof or peptides, which are necessary for the dissemination via the blood, but do not significantly affect the intestinal or mucosal colonization of pathogenic bacteria such as *E.coli, Salmonella typhimurium, Klebsiella pneumoniae, Yersinia pestis, Serratia marcescens, Haemophilus influenzae, Pasteurella multocida, Vibrio cholerae, Pseudomonas aeruginosa*, *Acetinobacter, Moraxella catarrhalis, Burkholderia pseudomallei, Neisseria meningitidis, Neisseria gonorrhoeae, Campylobacter jejuni, Helicobacter pylori, Bacteroides fragilis.*

In a process according to the invention, said nucleic acids, expression products or peptides are *E.coli* nucleic acids, expression products or peptides, respectively.

Pathogenicity or virulence targets encoded by isolated or purified nucleic acids having sequences SEQ ID Nos 16-30 have been obtained.

Pathogenicity or virulence factors corresponding to isolated or purified polypeptides or peptides having one of the amino acid sequences SEQ ID Nos 1-15 have been obtained.

These nucleic acids and peptides or polypeptides constitute targets for identifying compounds with a specific inhibitory effect on the systemic dissemination of a bacterial infection, and not on mucosal colonization or, for enterobacteria, on intestinal colonization, which makes it possible to preserve the commensal flora and to avoid the selection of resistance to the compounds.

The invention is thus directed towards a method for screening inhibitors of the expression products or peptides of pathogenic bacteria, comprising bringing into contact an isolated or purified nucleic acid of SEQ ID N°16, 20, 27 or 28 or the expression products thereof, or an isolated or purified peptide of SEQ ID N°1, 5, 12 or 13 with the test compound, detecting the possible effect of the compound on the expression products or peptides activity, and selecting the compounds having an inhibitory activity on virulence.

It is also directed towards a method for screening compounds capable of inhibiting the biochemical and/or enzyme activity of the polypeptides and peptides expressed by said nucleic acids.

Said method for screening inhibitors of the biochemical and/or enzyme activity of the expression products or peptides used, comprising bringing into contact said expression products or peptides with the test compound, detecting the possible effect of the compound on the peptides activity on virulence, and selecting the active compounds.

It is also directed to a method for use in the composition of an antibacterial product preventing dissemination of the infection in the host via the blood comprising bringing into contact the peptides used of SEQ ID N°1 1, 5, 12 or 13 with the candidate compound, detecting the possible effect of the compound on the expression products or peptides activity on virulence, and selecting the active compound.

The invention also relates to the use of SEQ ID N° 1, 5, 12 or 13 in a method to inhibit virulence of bacteria comprising,
- measuring the enzyme activity of said peptide,
- testing the multiplication of the bacteria in presence of serum and decomplemented serum,
- determining the virulence and proliferation of bacteria in an *in vivo* model of infection and determining the effect on gut colonisation in an *in vivo* mouse axenic model.

The invention thus provides a novel strategy and novel means for preventing or treating systemic bacterial dissemination, bacteraemia and septicaemia.

Other characteristics and advantages of the invention will be given in the following examples, with reference to Figures to 3 and tables 1 to 5, said figures representing, respectively,
- Figure 1, the growth of S26 and of the mutant pg23 in serum,
- Figure 2, the growth of S26 and of the mutant pg23 in decomplemented serum,and

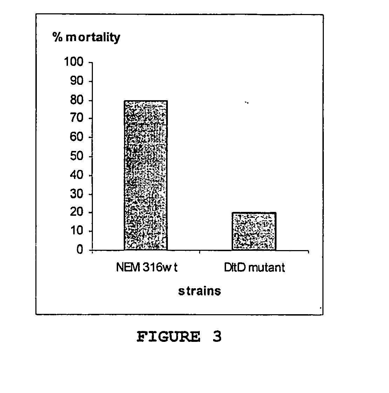

- Figure 3, the virulence of the DltD mutant of *S.agalactiae.*

### Example 1 gene corresponding to SEQ ID N°23:

### 1- Inactivation of the gene of interest

The general strategy, based on a recombination system, consists in interrupting a gene, by allelic recombination, with a gene for selection (a gene for resistance to antibiotic in the present case) carried by a linear DNA fragment.

Initially, a plasmid is introduced into the bacterium (for example *E.coli),* so as to introduce, in trans, the proteins which will induce the recombination. The plasmid carrying an ampicillin resistance gene is thermosensitive (30°C), which will make it possible to easily eliminate it after use in the bacterium.

The plasmid is introduced into the bacterium by electroporation. After electroporation, the ampicillin-resistant bacteria will be those which have integrated the plasmid, and will be selected. This step is entirely carried out at 30°C, the permissive temperature for the plasmid.

### Synthesis of the PCR fragment specific for the target gene (pg23)

A PCR is carried out, on a matrix plasmid carrying the selection gene (chloramphenicol resistance), using primers pg23P1 and pg23P2 of sequences SEQ ID No 31 and SEQ ID No 32, respectively, made up of two parts:
in 3': 20 bp homologous to the selection gene (chloramphenicol resistance): P1 or P2
in 5': 40 bp homologous to the target gene (*pg23*): H1 or H2

A DNA fragment consisting of the selection gene (CAT: Chloramphenicol Acetyl Transferase) flanked by the regions homologous to the target gene H1 and H2 is thus obtained.

### Step for inactivation of the target gene

The bacterium containing the plasmid is cultured in LB medium at 30°C with shaking, in the presence of 100 mM ampicillin and of 1 mM L-arabinose so as to induce the recombination system. When the bacteria are in the exponential growth phase (OD₆₀₀ₙₘ=0.5), the culture is stopped, and the bacteria are harvested and made electrocompetent. The PCR product specific for the target gene (*pg23)* is introduced into the bacterium by electroporation. The bacteria are then cultured in a non-selective rich medium (SOC medium) at 37°C with shaking for 2 hours, and then plated out onto selective LB agar medium. After 18 hours at 37°C, only the bacteria which have integrated the gene for resistance to the antibiotic will have grown.

### Verification of the insertion of the resistance cassette

In order to verify the insertion of the resistance cassette, PCR reactions are carried out directly using colonies. Three pairs of primers are used: a pair in which the primers FR1 and FR2 frame the target gene, and two pairs using a primer located inside the resistance cassette, the other primer being located either upstream or downstream of the target gene.

### Isolation of the mutated bacteria and elimination of the plasmid

The colonies thus verified by PCR are successively re-isolated on selected medium, twice on non-selective medium and a final time on selective medium at 37°C. Finally, the selected bacteria are tested for sensitivity to ampicillin, which reflects the absence of the plasmid. Three clones are thus chosen for each type of mutant.

### 2 - Test for the mutant with respect to resistance to the bactericidal activity of serum

The serum used is of human origin. In each experiment, growth was also effected for the wild-type strain (S26, clinical isolate of *E.coli* particularly resistant to serum and virulent in mice) and a strain, ECOR4, lacking a capsule and lipopolysaccharide (LPS). The growths were effected in triplicate and in two different sera. The growths were effected in parallel in complemented and decomplemented (30 min at 56°C) serum in order to verify that the effect observed was due only to the lytic action of complement.

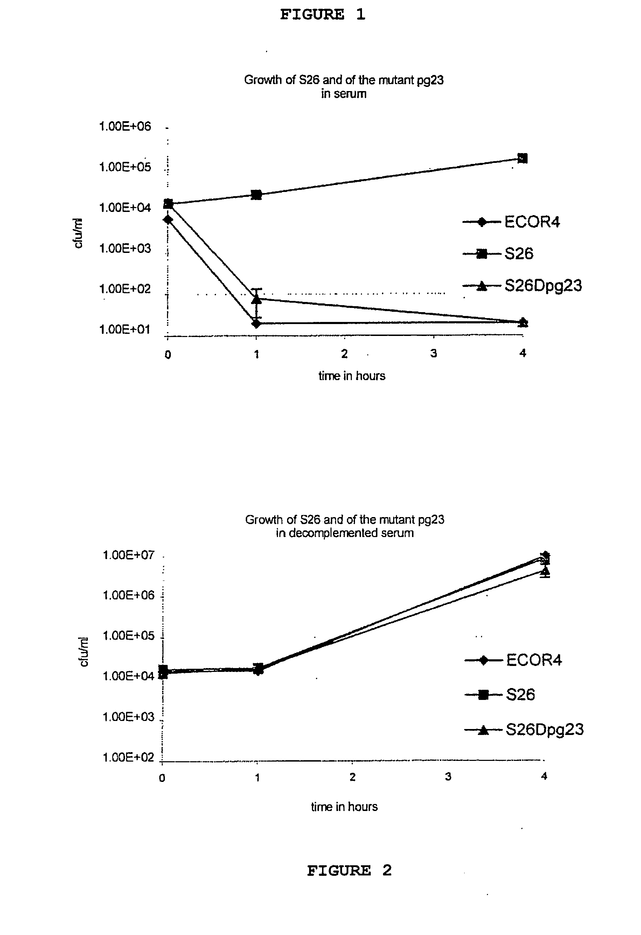

Using a preculture of two hours in RPMI reference minimum medium, the bacteria are brought into contact with 100% serum, at a starting inoculum of 10⁴cfu/ml. Counts are then performed at times 0, 1 and 4 hours, by plating various dilutions out on LB agar medium in the presence or absence of antibiotic. After 18 hours at 37°C, the bacteria are counted and a growth curve is produced from the results obtained. These results are given in Figures 1 and 2.

In this example, the mutant Δpg23 exhibits considerable sensitivity to the serum: a difference from the wild-type strain of more than 2 log at 1 hour and of more than 4 log at 4 hours is in fact observed. In addition, the results obtained in decomplemented serum and with the strain ECOR4 in serum indicate that the effect observed is indeed due to the bactericidal action of complement.

### 3 - Study of the virulence in a mouse animal model

### Preparation of the inoculum

The wild-type mutated bacteria are isolated from the strain, stored at -80°C, on an LB agar dish with or without antibiotic, and incubated at 37°C for 18 hours. A preculture is prepared in liquid medium. Using a 1/10th dilution in 10 ml of LB, the culture is regrown at 37°C with shaking for 2 hours. After culturing for 2 hours, the OD₆₀₀ₙₘ is measured and various dilutions are prepared in physiological saline, so as to obtain the desired inoculum. For the wild-type strain S26, the LD₅₀ corresponds to an inoculum of 5×10⁵ cfu/mouse and the LD₁₀₀ corresponds to an inoculum of 1×10⁶ cfu/mouse.

### Virulence test

The mice (6-week-old Balb/c) are given an intraperitoneal injection and the bacterial solution injected represents a volume of 100 µl. Five mice are used per dose. For S26Δpg23, 4 inoculums were tested and the survival rate was measured after 24 and 48 hours post-infection. In each experiment, the study was carried out in parallel with the wild-type strain, the LD₅₀ of which is 5×10⁵ cfu/mouse.

The mutant S26Δpg23, injected at a dose equal to 10 times the LD₁₀₀, causes no mortality, the mutation of the *pg23* gene in the *E.coli* strain K1 S26 is therefore responsible for a considerable decrease in the virulence.

### 4 - Study of the intestinal colonization in an axenic mouse animal model

The entire experiment is carried out in a sterile environment, with sterile instruments, in an isolator, and the mice are given sterile food.

### Mice

These are 6- to 8-week-old axenic female mice of the C3H/He J line.

Four animals are used per bacterial strain.

### Preparation of the inoculum

The wild-type and mutated bacteria are isolated from the strain, stored at -80°C, on an LB agar dish with or without antibiotic, and incubated at 37°C for 18 hours. After culturing the strain in liquid medium, various dilutions are prepared in physiological saline, so as to obtain an inoculum of 10⁷ cfu/ml.

### Colonization test

The bacterial inoculation is carried out orally. During the 24 hours preceding inoculation, the mice are deprived of water. They are then given a bacterial solution at 10⁷ cfu/ml to drink for 4 hours. The volume of drink is measured at 0 and 4 hours, and, on average, a mouse absorbs 5 ml of this bacterial solution. The faeces are then sampled at various times, and a bacterial count is performed, taking the faeces up in physiological saline and plating out various dilutions on an LB agar dish with and without antibiotic.
The results are given in table 1 herein below.

**TABLE 1**

| | CFU/mg faeces | |
|---|---|---|
| Time in hours | S26wt | S26Δpg23 |
| 0 | 0 | 0 |
| 4 | 6.85E+05 | 1.65E+05 |
| 25 | 1.86E+06 | 2.84E+06 |
| 118 | 8.34E+06 | 7.94E+06 |
| 456 | 4.14E+06 | 6.64E+06 |

For the wild-type strain S26, as well as for the mutant S26Δpg23, colonization in the intestine was stably established. No difference is observed between the wild-type strain and the mutant Δpg23. The colonization is confirmed on the final day by removing the intestine and counting the bacteria after grinding of this organ.

### 5 - Cloning and expression of the selected polypeptide

The nucleic acid encoding the polypeptide is cloned into a prokaryotic expression vector such as pET-14b with an N-terminal poly-his tag, according to conventional cloning methods.

The recombinant plasmid is then used to transform the E.coli strain BL21. The transformed cells are selected in the presence of ampicillin and the colonies are isolated. They are then cultured in the presence of IPTG in order to induce expression of the protein. The clones producing the protein are cultured and the total proteins are extracted by cell lysis. The recombinant protein is purified with a histidine tag affinity column, according to the manufacturer's protocol.

The protein thus obtained is purified and used *in vitro* to measure its enzyme activity.

### Example 2 serum sensitivity and LD₅₀ determination of mutant strains in the mice model of infection

Said mutants were also compared to the wild type S26 *E.coli* strain for LD₅₀ determination in the mice model of infection.
As presented in Table 2 below, the number of colony forming unit (cfu) counted after culture for four hours in serum was higher in the wild type (wt) S26 strain than in mutants indicating that mutants were sensitive to serum killing.
All the different mutants were either much less virulent in mice than the wild type strain as shown by the increase in LD₅₀ (lethal dose 50), or completely avirulent as no dose killing 50% of mice could be reach with the mutants.

**Table 2**

| Serum sensitivity and virulence attenuation for *E. coli* K1 S26 mutants in the proteins corresponding to sequence number 1 to 13 | | |
|---|---|---|
| Sequence Number | Serum sensitivity #Δlog (cfu/ml serum) | Virulence attenuation *Δlog (LD50) |
| 1 | +4 | avirulent^{a} |
| 2 | +4 | +1 |
| 3 | +5 | +1 |
| 4 | +4 | +1 |
| 5 | +4 | +2.5 |
| 6 | +4 | +0.5 |
| 7 | +4 | +0.5 |
| 8 | +4 | avirulent^{a} |
| 9 | +1 | avirulent^{a} |
| 10 | +2 | avirulent^{a} |
| 11 | +4 | +2 |
| 12 | +4 | +2 |
| 13 | +4 | avirulent^{a} |

| | | |
|---|---|---|
| avirulent^{a}: no dose killing 50% of mice could be reach with that mutant. # Δlog (cfu/ml serum) = log (cfu S26wt / ml serum) - log (cfu S26 mutant / ml serum) values obtained after 4 hours in serum *Δlog (LD₅₀) = log (LD₅₀ S26mutant) - log (LD₅₀ S26wt) values obtained 48 hours after inoculation | | |

The mutants of genes encoding the target proteins corresponding to sequences 1 to 13, which were attenuated for virulence, were still able to colonize the intestine of axenic mice as shown by persistence of bacteria in the faeces of the animals over a period of eight days. These results are presented in Table 3.

**Table 3**

| Gut colonization for *E. coli* K1 S26 wt and mutants in the proteins corresponding to sequence number 1 to 13 in an axenic mouse model | | | |
|---|---|---|---|
| | Sequence number | Gut colonization | |
| | | cfu/mg faeces | |
| | | Day 1 | Day 8 |
| S26 wt | | * 1.34x10⁶ | * 5.29x10⁶ |
| S26 mutants | 1 | 9.73x10⁵ | 2.51x10⁶ |
| | 2 | 1.02x10⁶ | 6.85x10⁶ |
| | 3 | 1.44x10⁶ | 3.48x10⁶ |
| | 4 | 1.24x10⁶ | 1.6x10⁶ |
| | 5 | 1.15x10⁵ | 4.64x10⁵ |
| | 6 | 9.96x10⁵ | 3.51x10⁶ |
| | 7 | 2.40x10⁴ | 2.51x10⁶ |
| | 8 | 2.84x10⁶ | 6.64x10⁶ |
| | 9 | 1.80x10⁶ | 1.51x10⁶ |
| | 10 | 9.62x10⁵ | 2.24x10⁶ |
| | 11 | 2.72x10⁵ | 8.56x10⁵ |
| | 12 | 3.13x10⁵ | 9.09x10⁵ |
| | 13 | 5.91x10⁵ | 1.67x10⁶ |

| | | | |
|---|---|---|---|
| * mean values based upon six experiments | | | |

The bacteria colonizing the intestine of axenic mice after eight days were characterized to verify that they correspond to the mutant strains that were inoculated orally.

The bacteria recovered from the faeces of animals had a phenotype of chloramphenicol resistance and serum sensitivity, the chloramphenicol acetyl transferase gene inserted during the mutagenesis could also be detected by PCR.

Mutations in genes encoding target proteins (sequence number 1 to 13) were still present in bacteria colonizing the intestine of axenic mice as shown in Table 4.

**Table 4**

| Characterization of bacteria recovered from axenic mice after intestinal colonization by mutants in genes encoding protein sequence 1 to 13 | | |
|---|---|---|
| Sequence | Serum sensitivity | * Mutant |
| Number | # ΔLog (cfu/ml serum) | genotype |
| 1 | +5 | Cm^{R}, PCR + |
| 2 | +4 | Cm^{R} |
| 3 | + 5 | Cm^{R} |
| 4 | + 3 | Cm^{R} |
| 5 | +5 | Cm^{R}, PCR + |
| 6 | +2 | Cm^{R} |
| 7 | +2 | Cm^{R} |
| 8 | Nd | Cm^{R} |
| 9 | +2 | Cm^{R} |
| 10 | +3 | cm^{R} |
| 11 | +5 | Cm^{R} PCR + |
| 12 | +4 | Cm^{R} PCR + |
| 13 | +4 | Cm^{R}, PCR + |

| | | |
|---|---|---|
| # ΔLog (cfu/ml serum) = log (cfu S26wt / ml serum) - log (cfu S26mutant / ml serum) values obtained after 4 hours in serum *The presence of the gene encoding the chloramphenicol acetyltransferase, inactivating the genes encoding the proteins of sequence number 1 to 13, has been verified by PCR and chloramphenicol resistance (Cm^{R}). | | |

In conclusion, the results presented in this example demonstrate that genes encoding the enzymes involved in the LPS inner core metabolism are not essential in *E.coli* strains for colonization, but are necessary for resistance to complement and virulence *in vivo*.

They represent as such good targets for inhibitors that will selectively block bacterial replication in blood tissue.

### Example 2: mutants of protein SEQ ID N°14

The present invention relates to novel mutant strain of Group B Streptococcus (GBS) *(Streptococcus agalactiae*). In this particular example, the identified targets correspond to gene sequence number 29 encoding a protein sequence number 14 involved in incorporation of D-alanine residues into the cell wall-associated lipoteichoic acids (LTAs) in Gram + bacteria.

The gene sequence number 29 is homologous to the *dltD* gene found in other gram positive bacteria and is the last gene of the dlt operon.

The Gram + bacterial model used is the pathogenic strain *S*. *agalactiae* NEM316. *S. agalactiae* is a bacterium commonly found in the human flora and is phylogeneticaly close to Gram + bacteria responsible for nosocomial septicemia.

The virulence of GBS mutants in the dlt operon is strongly impaired in mouse and newborn rat models.

Interestingly, the loss of virulence is presumably due to an increased sensitivity to antimicrobial cationic peptides, such as defensins, which are produced by numerous cells types in particular phagocytes.

The use of mutant of *S*. *agalactiae,* in which the *dltD* gene have been inactivated, demonstrates that the product of that gene is a good target for the development of inhibitors of virulence of *S*. *agalactiae* as well as against other Gram + pathogens.

## Claims

1. A method for identifying and selecting virulence gene targets in pathogenic bacteria, comprising the following steps:
- using a strain of the pathogenic bacteria,
- generating mutant strains for inactivation of genes encoding virulence factors,
- determining the virulence of the mutated bacteria on an experimental model of infection, and their effect on enteric colonization in an axenic mouse model, and
- identifying and selecting the bacteria having a mutation in genes that prove to have all the following features: genes non essential for proliferation *in vitro,* non essential for the commensal colonization, but essential for resistance to serum *in vitro*, and essential, in the host, for dissemination in blood.

2. The method of claim 1, comprising the step of assessing the viability of the mutant bacteria *in vitro* and its ability to resist and proliferate in presence of complemented serum.

3. The method of claim 1 or 2, wherein the pathogenic bacteria are EXPEC *E.coli* strains.

4. *In vitro* process for inhibiting the virulence of pathogenic bacteria, comprising targeting with an inhibitor a nucleic acid of SEQ ID N°16, 20, 27 or 28 or a peptide of SEQ ID N°1, 5, 12 or 13, to increase the serum sensibility of the bacteria and attenuate their virulence.

5. The process according to claim 4, wherein said nucleic acids, expression products thereof or peptides correspond to those of pathogenic organisms, comprising *Escherichia coli, Salmonella typhimurium, Klebsiella pneumoniae*, *Yersinia pestis, Serratia marcescens, Haemophilus influenzae, Pasteurella multocida, Vibrio cholerae, Pseudomonas aeruginosa*, *Acetinobacter, Moraxella catarrhalis, Burkholderia pseudomallei*, *Neisseria meningitidis, Neisseria gonorrhoeae, Campylobacter jejuni, Helicobacter pylori, Bacteroides fragilis.*

6. The process of claim 5, wherein said nucleic acids, expression products or peptides are *E.coli* nucleic acids, expression products or peptides, respectively.

7. A method for screening inhibitors of the expression products or peptides of pathogenic bacteria, comprising bringing into contact an isolated or purified nucleic acid of SEQ ID N°16, 20, 27 or 28 or the expression products thereof, or an isolated or purified peptide of SEQ ID N°1, 5, 12 or 13 with the test compound, detecting the possible effect of the compound on the expression products or peptides activity, and selecting the compounds having an inhibitory activity on virulence.

8. A method for screening inhibitors of the biochemical and/or enzyme activity of the expression products or peptides used according to claim 4, comprising bringing into contact said expression products or peptides with the test compound, detecting the possible effect of the compound on the peptides activity on virulence, and selecting the active compounds.

9. A method for identifying a candidate compound for use in the composition of an antibacterial product preventing dissemination of the infection in the host via the blood comprising bringing into contact the peptides used according to claim 4 with the candidate compound, detecting the possible effect of the compound on the expression products or peptides activity on virulence, and selecting the active compound.

10. Use of SEQ ID N° 1, 5, 12 or 13 in a method to inhibit virulence of bacteria comprising,
- measuring the enzyme activity of said peptide,
- testing the multiplication of the bacteria in presence of serum and decomplemented serum,
- determining the virulence and proliferation of bacteria in an *in vivo* model of infection and determining the effect on gut colonisation in an *in vivo* mouse axenic model.

## Patentansprüche

1. Verfahren zum Identifizieren und Selektieren von Virulenz-Gen-Targets in pathogenen Bakterien, das die folgenden Schritte umfasst:
- Verwenden eines Stamms der pathogenen Bakterien;
- Erzeugen von mutanten Stämmen für die Inaktivierung von Genen, die Virulenzfaktoren codieren;
- Bestimmen der Virulenz der mutierten Bakterien an einem experimentellen Modell der Infektion und ihrer Wirkung auf die Darmbesiedlung in einem axenischen Mausmodell; und
- Identifizieren und Selektieren der Bakterien, die eine Mutation in Genen aufweisen, von denen sich erweist, dass sie alle der folgenden Merkmale haben: Gene, die für die Proliferation in vitro nicht wesentlich und für die kommensale Besiedlung nicht wesentlich sind, aber für die Resistenz gegen Serum in vitro wesentlich sind und im Wirt für die Verbreitung im Blut wesentlich sind.

2. Verfahren gemäß Anspruch 1, das den Schritt des Bewertens der Lebensfähigkeit der mutanten Bakterien in vitro und deren Fähigkeit, in Gegenwart von komplementiertem Serum resistent zu sein und sich zu vermehren, umfasst.

3. Verfahren gemäß Anspruch 1 oder 2, wobei die pathogenen Bakterien EXPEC-E. -coli-Stämme sind.

4. In-vitro-Verfahren zur Hemmung der Virulenz von pathogenen Bakterien, das die Zielsteuerung mit einem Inhibitor einer Nucleinsäure von SEQ ID Nr. 16, 20, 27 oder 28 oder eines Peptids von SEQ ID Nr. 1, 5, 12 oder 13 umfasst, um die Serumempfindlichkeit der Bakterien zu erhöhen und ihre Virulenz abzuschwächen.

5. Verfahren gemäß Anspruch 4, wobei die Nucleinsäuren, Expressionsprodukte davon oder Peptide denjenigen von pathogenen Organismen entsprechen, die *Escherichia coli, Salmonella typhimurium, Klebsiella pneumoniae, Yersinia pestis, Serratia marcescens, Haemophilus influenzae, Pasteurella multocida, Vibrio cholerae, Pseudomonas aeruginosa, Acetinobacter, Moraxella catarrhalis, Burkholderia pseudomallei, Neisseria meningitidis, Neisseria gonorrhoeae, Campylobacter jejuni, Helicobacter pylori, Bacteroides fragilis* umfassen.

6. Verfahren gemäß Anspruch 5, wobei die Nucleinsäuren, Expressionsprodukte oder Peptide *E.-coli*-Nucleinsäuren, -Expressionsprodukte bzw. -Peptide sind.

7. Verfahren zur Suche nach Inhibitoren der Expressionsprodukte oder Peptide von pathogenen Bakterien, umfassend das In-Kontakt-Bringen einer isolierten oder gereinigten Nucleinsäure von SEQ ID Nr. 16, 20, 27 oder 28 oder ihrer Expressionsprodukte oder eines isolierten oder gereinigten Peptids von SEQ ID Nr. 1, 5, 12 oder 13 mit der Testverbindung, Nachweisen der möglichen Wirkung der Verbindung auf die Aktivität der Expressionsprodukte oder Peptide und Auswählen der Verbindungen, die eine inhibitorische Aktivität auf die Virulenz aufweisen.

8. Verfahren zur Suche nach Inhibitoren der biochemischen und/oder Enzymaktivität der gemäß Anspruch 4 verwendeten Expressionsprodukte oder Peptide, umfassend das In-Kontakt-Bringen der Expressionsprodukte oder Peptide mit der Testverbindung, Nachweisen der möglichen Wirkung der Verbindung auf die Aktivität, die die Peptide auf die Virulenz haben, und Auswählen der aktiven Verbindungen.

9. Verfahren zum Identifizieren einer Verbindung, die für die Verwendung in der Zusammensetzung eines antibakteriellen Produkts zur Verhinderung der Ausbreitung der Infektion im Wirt über das Blut in Frage kommt, umfassend das In-Kontakt-Bringen der gemäß Anspruch 4 verwendeten Peptide mit der in Frage kommenden Verbindung, Nachweisen der möglichen Wirkung der Verbindung auf die Aktivität, die die Expressionsprodukte oder Peptide auf die Virulenz haben, und Auswählen der aktiven Verbindung.

10. Verwendung von SEQ ID Nr. 1, 5, 12 oder 13 in einem Verfahren zur Hemmung der Virulenz von Bakterien, umfassend:
- Messen der Enzymaktivität auf das Peptid;
- Testen der Vermehrung der Bakterien in Gegenwart von Serum und dekomplementiertem Serum;
- Bestimmen der Virulenz und Proliferation von Bakterien in einem in-vivo-Modell der Infektion und Bestimmen der Wirkung auf die Darmbesiedlung in einem axenischen in-vivo-Mausmodell.

## Revendications

1. Méthode pour identifier et sélectionner des cibles de gènes dans des bactéries pathogènes, comprenant les étapes suivantes:
- l'utilisation d'une souche des bactéries pathogènes,
- la génération de souches mutantes pour l'inactivation de gènes codant pour des facteurs de virulence,
- la détermination de la virulence des bactéries mutées sur un modèle expérimental d'infection, et de leur effet sur la colonisation entérique chez un modèle de souris axénique, et
- l'identification et la sélection des bactéries ayant une mutation dans les gènes qui s'avèrent avoir toutes les caractéristiques suivantes : gènes non essentiels pour la prolifération *in vitro,* non essentiels pour la colonisation commensale, mais essentiels pour la résistance au sérum in vitro, et essentiels, chez l'hôte, pour la dissémination dans le sang.

2. Méthode selon la revendication 1, **caractérisée en ce qu'**elle comprend l'étape de détermination de la viabilité des bactéries mutantes *in vitro* et de leur capacité à résister et à proliférer en présence de sérum complémenté.

3. Méthode selon la revendication 1 ou 2, **caractérisée en ce que** les bactéries pathogènes sont des souches EXPEC de *E.coli.*

4. Méthode *in vitro* pour inhiber la virulence de bactéries pathogènes, **caractérisée en ce qu'**elle comprend le ciblage avec un inhibiteur d'un acide nucléique de SEQ ID N°16, 20, 27 ou 28 ou un peptide de SEQ ID N°1, 5, 12 ou 13, pour augmenter la sensibilité au sérum des bactéries et atténuer leur virulence.

5. Méthode selon la revendication 4, **caractérisée en ce** lesdits acides nucléiques, leurs produits d'expression ou les peptides correspondent à ceux d'organismes pathogènes comprenant *E.coli, Salmonella typhimurium* , *Klebsiella pneumoniae, Yersinia pestis, Serratia marcescens, Haemophilus influenzae, Pasteurella multocida, Vibrio cholerae, Pseudomonas aeruginosa, Acetinobacter, Moraxella catarrhalis, Burkholderia pseudomallei, Neisseria meningitidis, Neisseria gonorrhoeae, Campylobacter jejuni, Helicobacter pylori, Bacteroides fragilis.*

6. Méthode selon la revendication 5, **caractérisée en ce que** lesdits acides nucléiques, les produits d'expression ou les peptides sont, respectivement, des acides nucléiques, des produits d'expression ou des peptides de *E.coli.*

7. Méthode de criblage d'inhibiteurs des produits d'expression ou des peptides de bactéries pathogènes, **caractérisée en ce qu'**elle comprend la mise en contact d'un acide nucléique isolé ou purifié de SEQ ID N°16, 20, 27 ou 28 ses produits d'expression, ou un peptide isolé ou purifié de SEQ ID N°1, 5, 12 ou 13 avec le composé à tester, la détection de l'effet éventuel du composé sur l'activité des produits d'expression ou des peptides, et la sélection des composés actifs ayant une activité inhibitrice sur la virulence.

8. Méthode de criblage d'inhibiteurs de l'activité biochimique et/ou enzymatique des produits d'expression ou des peptides utilisés selon la revendication 4, **caractérisée en ce qu'**elle comprend la mise en contact des produits d'expression ou des peptides avec le composé à tester, la détection d'un éventuel effet du composé sur l'activité des peptides sur la virulence, et la sélection des composés actifs.

9. Méthode pour identifier un composé candidat utilisable dans la composition d'un produit antibactérien empêchant la dissémination de l'infection chez l'hôte *via* le sang, **caractérisée en ce qu'**elle comprend la mise en contact des peptides utilisés selon la revendication 4, avec le composé candidat, la détection de l'effet éventuel du composé sur l'activité sur la virulence des produits d'expression ou des peptides, et la sélection du composé actif.

10. Utilisation de SEQ ID N°1, 5, 12 ou 13 dans un procédé pour inhiber la virulence de bactéries, **caractérisée en ce qu'**elle comprend,
- la mesure de l'activité de l'enzyme dudit peptide,
- l'évaluation de la multiplication des bactéries en présence de sérum et de sérum décomplémenté,
- la détermination de la virulence et de la prolifération de bactéries dans un modèle *in vivo* d'infection et la détermination de l'effet sur la colonisation de l'intestin dans un modèle de souris axénique *in vivo.*


### SEQUENCE LISTING

<110> MUTABILIS S.A. can be used as
   <120> Pathogenicity determinants which can be used as targets for developing means for preventing and controlling bacterial infections and/or systemic dissemination
   <130> 1621
   <160> 32
   <170> Patent In version 3.1
   <210> 1
   <211> 305
   <212> PRT
   <213> Escherichia coli
   <400> 1
   Pro Ala Leu Thr Asp Ala Gln Gln Ala Ile Pro Gly Ile Lys Phe Asp
   1               5                   10                  15
   Trp Val Val Glu Glu Gly Phe Ala Gln Ile Pro Ser Trp His Ala Ala
               20                  25                  30
   Val Glu Arg Val Ile Pro Val Ala Ile Arg Arg Trp Arg Lys Ala Trp
           35                  40                  45
   Phe Ser Ala Pro Ile Lys Ala Glu Arg Lys Ala Phe Arg Glu Ala Leu
       50                  55                  60
   Gln Ala Glu Asn Tyr Asp Ala Val Ile Asp Ala Gln Gly Leu Val Lys
   65                  70                  75                  80
   Ser Ala Ala Leu Val Thr Arg Leu Ala His Gly Val Lys His Gly Leu
                   85                  90                  95
   Asp Trp Gln Thr Ala Arg Glu Pro Leu Ala Ser Leu Phe Tyr Asn Cys
               100                 105                 110
   Lys His His Ile Ala Lys Gln Gln His Ala Val Glu Arg Thr Arg Glu
           115                 120                 125
   Leu Phe Ala Lys Ser Leu Gly Tyr Ser Lys Pro Gln Thr Gln Gly Asp
       130                 135                 140
   Tyr Ala Ile Ala Gln His Phe Leu Thr Asn Leu Pro Thr Asp Ala Gly
   145                 150                 155                 160
   Glu Tyr Ala Val Phe Leu His Ala Thr Thr Arg Asp Asp Lys His Trp
                   165                 170                 175
   Pro Glu Glu His Trp Arg Glu Leu Ile Gly Leu Leu Ala Asp Ser Gly
               180                 185                 190
   Ile Arg Ile Lys Leu Pro Trp Gly Ala Pro His Glu Glu Glu Arg Ala
   Lys Arg Leu Ala Glu Gly Phe Ala Tyr Val Glu Val Leu Pro Lys Met
       210                 215                 220
   Ser Leu Glu Gly Val Ala Arg Val Leu Ala Gly Ala Lys Phe Val Val
   225                 230                 235                 240
   Ser Val Asp Thr Gly Leu Ser His Leu Thr Ala Ala Leu Asp Arg Pro
                   245                 250                 255
   Asn Ile Thr Val Tyr Gly Pro Thr Asp Pro Gly Leu Ile Gly Gly Tyr
               260                 265                 270
   Gly Lys Asn Gln Met Val Cys Arg Ala Pro Gly Asn Glu Leu Ser Gln
           275                 280                 285
   Leu Thr Ala Asn Ala Val Lys Arg Phe Ile Glu Glu Asn Ala Ala Met
       290                 295                 300
   Ile
   305
<210> 2
   <211> 340
   <212> PRT
   <213> Escherichia coli
   <400> 2
   Met Arg Phe His Gly Asp Met Leu Leu Thr Thr Pro Val Ile Ser Ser
   1               5                   10                  15
   Leu Lys Lys Asn Tyr Pro Asp Ala Lys Ile Asp Val Leu Leu Tyr Gln
               20                  25                  30
   Asp Thr Ile Pro Ile Leu Ser Glu Asn Pro Glu Ile Asn Ala Leu Tyr
           35                  40                  45
   Gly Ile Lys Asn Lys Lys Ala Lys Ala Ser Glu Lys Ile Ala Asn Phe
       50                  55                  60
   Phe His Leu Ile Lys Val Leu Arg Ala Asn Lys Tyr Asp Leu Ile Val
   65                  70                  75                  80
   Asn Leu Thr Asp Gln Trp Met Val Ala Ile Leu Val Arg Leu Leu Asn
                   85                  90                  95
   Ala Arg Val Lys Ile Ser Gln Asp Tyr His His Arg Gln Ser Ala Phe
               100                 105                 110
   Trp Arg Lys Ser Phe Thr His Leu Val Pro Leu Gln Gly Gly Asn Val
           115                 120                 125
   Val Glu Ser Asn Leu Ser Val Leu Thr Pro Leu Gly Val Asp Ser Leu
       130                 135                 140
   Val Lys Gln Thr Thr Met Ser Tyr Pro Pro Ala Ser Trp Lys Arg Met
   145                 150                 155                 160
   Arg Arg Glu Leu Asp His Ala Gly Val Gly Gln Asn Tyr Val Val Ile
                   165                 170                 175
   Gln Pro Thr Ala Arg Gln Ile Phe Lys Cys Trp Asp Asn Ala Lys Phe
               180                 185                 190
   Ser Ala Val Ile Asp Ala Leu His Ala Arg Gly Tyr Glu Val Val Leu
           195                 200                 205
   Thr Ser Gly Pro Asp Lys Asp Asp Leu Ala Cys Val Asn Glu Ile Ala
       210                 215                 220
   Gln Gly Cys Gln Thr Pro Pro Val Thr Ala Leu Ala Gly Lys Val Thr
   225                 230                 235                 240
   Phe Pro Glu Leu Gly Ala Leu Ile Asp His Ala Gln Leu Phe Ile Gly
                   245                 250                 255
   Val Asp Ser Ala Pro Ala His Ile Ala Ala Ala Val Asn Thr Pro Leu
               260                 265                 270
   Ile Ser Leu Phe Gly Ala Thr Asp His Ile Phe Trp Arg Pro Trp Ser
           275                 280                 285
   Asn Asn Met Ile Gln Phe Trp Ala Gly Asp Tyr Arg Glu Met Pro Thr
       290                 295                 300
   Arg Asp Gln Arg Asp Arg Asn Glu Met Tyr Leu Ser Val Ile Pro Ala
   305                 310                 315                 320
   Ala Asp Val Ile Ala Ala Val Asp Lys Leu Leu Pro Ser Ser Thr Thr
                   325                 330                 335
   Gly Thr Ser Leu
               340
<210> 3
   <211> 265
   <212> PRT
   <213> Escherichia coli
   <400> 3
   Met Val Glu Leu Lys Glu Pro Phe Ala Thr Leu Trp Arg Gly Lys Asp
   1               5                   10                  15
   Pro Phe Glu Glu Val Lys Thr Leu Gln Gly Glu Val Phe Arg Glu Leu
               20                  25                  30
   Glu Thr Arg Arg Thr Leu Arg Phe Glu Met Ala Gly Lys Ser Tyr Phe
           35                  40                  45
   Leu Lys Trp His Arg Gly Thr Thr Leu Lys Glu Ile Ile Lys Asn Leu
       50                  55                  60
   Leu Ser Leu Arg Met Pro Val Leu Gly Ala Asp Arg Glu Trp Asn Ala
   65                  70                  75                  80
   Ile His Arg Leu Arg Asp Val Gly Val Asp Thr Met Tyr Gly Val Ala
                   85                  90                  95
   Phe Gly Glu Lys Gly Met Asn Pro Leu Thr Arg Thr Ser Phe Ile Ile
               100                 105                 110
   Thr Glu Asp Leu Thr Pro Thr Ile Ser Leu Glu Asp Tyr Cys Ala Asp
           115                 120                 125
   Trp Ala Thr Asn Pro Pro Asp Val Arg Val Lys Arg Met Leu Ile Lys
       130                 135                 140
   Arg Val Ala Thr Met Val Arg Asp Met His Ala Ala Gly Ile Asn His
   145                 150                 155                 160
   Arg Asp Cys Tyr Ile Cys His Phe Leu Leu His Leu Pro Phe Ser Gly
                   165                 170                 175
   Lys Glu Glu Glu Leu Lys Ile Ser Val Ile Asp Leu His Arg Ala Gln
               180                 185                 190
   Leu Arg Thr Arg Val Pro Arg Arg Trp Arg Asp Lys Asp Leu Ile Gly
           195                 200                 205
   Leu Tyr Phe Ser Ser Met Asn Ile Gly Leu Thr Gln Arg Asp Ile Trp
       210                 215                 220
   Arg Phe Met Lys Val Tyr Phe Ala Ala Pro Leu Lys Asp Ile Leu Lys
   225                 230                 235                 240
   Gln Glu Gln Gly Leu Leu Ser Gln Ala Glu Ala Lys Ala Thr Lys Ile
                   245                 250                 255
   Arg Glu Arg Thr Ile Arg Lys Ser Leu
               260                 265
<210> 4
   <211> 374
   <212> PRT
   <213> Escherichia coli
   <400> 4
   Met Ile Val Ala Phe Cys Leu Tyr Lys Tyr Phe Pro Phe Gly Gly Leu
   1               5                   10                  15
   Gln Arg Asp Phe Met Arg Ile Ala Gln Thr Val Ala Ala Arg Gly His
               20                  25                  30
   His Val Arg Val Tyr Thr Gln Ser Trp Glu Gly Glu Cys Pro Asp Val
           35                  40                  45
   Phe Glu Leu Ile Lys Val Pro Val Lys Ser His Thr Asn His Gly Arg
       50                  55                  60
   Asn Ala Glu Tyr Phe Ala Trp Val Gln Lys His Leu Arg Glu His Pro
   65                  70                  75                  80
   Val Asp Lys Val Val Gly Phe Asn Lys Met Pro Gly Leu Asp Val Tyr
                   85                  90                  95
   Tyr Ala Ala Asp Val Cys Tyr Ala Glu Lys Val Ala Gln Glu Lys Gly
               100                 105                 110
   Phe Phe Tyr Arg Leu Thr Ser Arg Tyr Arg His Tyr Ala Ala Phe Glu
           115                 120                 125
   Arg Ala Thr Phe Glu Gln Gly Lys Pro Thr Gln Leu Leu Met Leu Thr
       130                 135                 140
   Asp Lys Gln Ile Ala Asp Phe Gln Lys His Tyr Gln Thr Glu Ala Glu
   145                 150                 155                 160
   Arg Phe His Ile Leu Pro Pro Gly Ile Tyr Pro Asp Arg Lys Tyr Ser
                   165                 170                 175
   Gln Gln Pro Ala Asn Ser Arg Glu Ile Phe Arg Lys Lys Asn Gly Ile
               180                 185                 190
   Thr Glu Gln Gln Tyr Leu Leu Leu Gln Val Gly Ser Asp Phe Thr Arg
           195                 200                 205
   Lys Gly Val Asp Arg Ser Ile Glu Ala Leu Ala Ser Leu Pro Asp Ser
       210                 215                 220
   Leu Arg His Asn Thr Leu Leu Tyr Val Val Gly Gln Asp Lys Pro Arg
   225                 230                 235                 240
   Lys Phe Glu Ala Leu Ala Glu Lys Arg Gly Val Arg Ser Asn Val His
   245 250 255
   Phe Phe Ser Gly Arg Asn Asp Val Ser Glu Leu Met Ala Ala Ala Asp
   260 265 270
   Leu Leu Leu His Pro Ala Tyr Gln Glu Ala Ala Gly Ile Val Leu Leu
   275 280 285
   Glu Ala Ile Thr Ala Gly Leu Pro Val Leu Thr Thr Ala Val Cys Gly
   290 295 300
   Tyr Ala His Tyr Ile Val Asp Ala Asn Cys Gly Glu Ala Ile Ala Glu
   305 310 315 320
   Pro Phe Arg Gln Glu Thr Leu Asn Glu Ile Leu Arg Lys Ala Leu Thr
   325 330 335
   Gln Ser Ser Leu Arg Gln Ala Trp Ala Glu Asn Ala Arg His Tyr Ala
   340 345 350
   Asp Thr Gln Asp Leu Tyr Ser Leu Pro Glu Lys Ala Ala Asp Ile Ile
   355 360 365
   Thr Gly Gly Leu Asp Gly
   370
<210> 5
   <211> 348
   <212> PRT
   <213> Escherichia coli
   <400> 5
   Met Lys Ile Leu Val Ile Gly Pro Ser Trp Val Gly Asp Met Met Met
   1               5                   10                  15
   Ser Gln Ser Leu Tyr Arg Thr Leu Gln Ala Arg Tyr Pro Gln Ala Ile
               20                  25                  30
   Ile Asp Val Met Ala Pro Ala Trp Cys Arg Pro Leu Leu Ser Arg Met
           35                  40                  45
   Pro Glu Val Asn Glu Ala Ile Pro Met Pro Leu Gly His Gly Ala Leu
       50                  55                  60
   Glu Ile Gly Glu Arg Arg Lys Leu Gly His Ser Leu Arg Glu Lys Arg
   65                  70                  75                  80
   Tyr Asp Arg Ala Tyr Val Leu Pro Asn Ser Phe Lys Ser Ala Leu Val
                   85                  90                  95
   Pro Phe Phe Ala Gly Ile Pro His Arg Thr Gly Trp Arg Gly Glu Met
                   100                 105                 110
   Arg Tyr Gly Leu Leu Asn Asp Val Arg Val Leu Asp Lys Glu Ala Trp
           115                 120                 125
   Pro Leu Met Val Glu Arg Tyr Ile Ala Leu Ala Tyr Asp Lys Gly Ile
       130                 135                 140
   Met Arg Thr Ala Gln Asp Leu Pro Gln Pro Leu Leu Trp Pro Gln Leu
   145                 150                 155                 160
   Gln Val Ser Glu Gly Glu Lys Ser Tyr Thr Cys Asn Gln Phe Ser Leu
                   165                 170                 175
   Ser Ser Glu Arg Pro Met Ile Gly Phe Cys Pro Gly Ala Glu Phe Gly
               180                 185                 190
   Pro Ala Lys Arg Trp Pro His Tyr His Tyr Ala Glu Leu Ala Lys Gln
           195                 200                 205
   Leu Ile Asp Glu Gly Tyr Gln Val Val Leu Phe Gly Ser Ala Lys Asp
       210                 215                 220
   His Glu Ala Gly Asn Glu Ile Leu Ala Ala Leu Asn Thr Glu Gln Gln
   225                 230                 235                 240
   Ala Trp Cys Arg Asn Leu Ala Gly Glu Thr Gln Leu Asp Gln Ala Val
                   245                 250                 255
   Ile Leu Ile Ala Ala Cys Lys Ala Ile Val Thr Asn Asp Ser Gly Leu
               260                 265                 270
   Met His Val Ala Ala Ala Leu Asn Arg Pro Leu Val Ala Leu Tyr Gly
           275                 280                 285
   Pro Ser Ser Pro Asp Phe Thr Pro Pro Leu Ser His Lys Ala Arg Val
       290                 295                 300
   Ile Arg Leu Ile Thr Gly Tyr His Lys Val Arg Lys Gly Asp Ala Ala
   305                 310                 315                 320
   Glu Gly Tyr His Gln Ser Leu Ile Asp Ile Thr Pro Gln Arg Val Leu
                   325                 330                 335
   Glu Glu Leu Asn Ala Leu Leu Leu Gln Glu Glu Ala
               340                 345
<210> 6
   <211> 338
   <212> PRT
   <213> Escherichia coli
   <400> 6
   Met Ser Ala His Tyr Phe Asn Pro Gln Glu Met Ile Asn Lys Thr Ile
   1               5                   10                  15
   Ile Phe Asp Glu Arg Pro Ala Ala Ser Val Ala Ser Ser Phe His Val
               20                  25                  30
   Ala Tyr Gly Ile Asp Lys Asn Phe Leu Phe Gly Cys Gly Val Ser Ile
           35                  40                  45
   Thr Ser Val Leu Leu His Asn Asn Asp Val Ser Phe Val Phe His Val
       50                  55                  60
   Phe Ile Asp Asp Ile Pro Glu Ala Asp Ile Gln Arg Leu Ala Gln Leu
   65                  70                  75                  80
   Ala Lys Ser Tyr Arg Thr Cys Ile Gln Ile His Leu Val Asn Cys Glu
                   85                  90                  95
   Arg Leu Lys Ala Leu Pro Thr Thr Lys Asn Trp Ser Ile Ala Met Tyr
               100                 105                 110
   Phe Arg Phe Val Ile Ala Asp Tyr Phe Ile Asp Gln Gln Asp Lys Ile
           115                 120                 125
   Leu Tyr Leu Asp Ala Asp Ile Ala Cys Gln Gly Asn Leu Lys Pro Leu
       130                 135                 140
   Ile Thr Met Asp Leu Ala Asn Asn Val Ala Ala Val Val Thr Glu Arg
   145                 150                 155                 160
   Asp Ala Asn Trp Trp Ser Leu Arg Gly Gln Ser Leu Gln Cys Asn Glu
                   165                 170                 175
   Leu Glu Lys Gly Tyr Phe Asn Ser Gly Val Leu Leu Ile Asn Thr Leu
               180                 185                 190
   Ala Trp Ala Gln Glu Ser Val Ser Ala Lys Ala Met Ser Met Leu Ala
           195                 200                 205
   Asp Lys Ala Ile Val Ser Arg Leu Thr Tyr Met Asp Gln Asp Ile Leu
       210                 215                 220
   Asn Leu Ile Leu Leu Gly Lys Val Lys Phe Ile Asp Ala Lys Tyr Asn
   225                 230                 235                 240
   Thr Gln Phe Ser Leu Asn Tyr Glu Leu Lys Lys Ser Phe Val Cys Pro
                   245                 250                 255
   Ile Asn Asp Glu Thr Val Leu Ile His Tyr Val Gly Pro Thr Lys Pro
   260 265 270
   Trp His Tyr Trp Ala Gly Tyr Pro Ser Ala Gln Pro Phe Ile Lys Ala
   275 280 285
   Lys Glu Ala Ser Pro Trp Lys Asn Glu Pro Leu Met Arg Pro Val Asn
   290 295 300
   Ser Asn Tyr Ala Arg Tyr Cys Ala Lys His Asn Phe Lys Gln Asn Lys
   305 310 315 320
   Pro Ile Asn Gly Ile Met Asn Tyr Ile Tyr Tyr Phe Tyr Leu Lys Ile
   325 330 335
   Ile Lys
<210> 7
   <211> 302
   <212> PRT
   <213> Escherichia coli
   <400> 7
   Met Ala Ala Ile Asn Thr Lys Val Lys Lys Ala Val Ile Pro Val Ala
   1               5                   10                  15
   Gly Leu Gly Thr Arg Met Leu Pro Ala Thr Lys Ala Ile Pro Lys Glu
               20                  25                  30
   Met Leu Pro Leu Val Asp Lys Pro Leu Ile Gln Tyr Val Val Asn Glu
           35                  40                  45
   Cys Ile Ala Ala Gly Ile Thr Glu Ile Val Leu Val Thr His Ser Ser
       50                  55                  60
   Lys Asn Ser Ile Glu Asn His Phe Asp Thr Ser Phe Glu Leu Glu Ala
   65                  70                  75                  80
   Met Leu Glu Lys Arg Val Lys Arg Gln Leu Leu Asp Glu Val Gln Ser
                   85                  90                  95
   Ile Cys Pro Pro His Val Thr Ile Met Gln Val Arg Gln Gly Leu Ala
               100                 105                 110
   Lys Gly Leu Gly His Ala Val Leu Cys Ala His Pro Val Val Gly Asp
           115                 120                 125
   Glu Pro Val Ala Val Ile Leu Pro Asp Val Ile Leu Asp Glu Tyr Glu
       130                 135                 140
   Ser Asp Leu Ser Gln Asp Asn Leu Ala Glu Met Ile Arg Arg Phe Asp
   145 150 155 160
   Glu Thr Gly His Ser Gln Ile Met Val Glu Pro Val Ala Asp Val Thr
   165 170 175
   Ala Tyr Gly Val Val Asp Cys Lys Gly Val Glu Leu Ala Pro Gly Glu
   180 185 190
   Ser Val Pro Met Val Gly Val Val Glu Lys Pro Lys Ala Asp Val Ala
   195 200 205
   Pro Ser Asn Leu Ala Ile Val Gly Arg Tyr Val Leu Ser Ala Asp Ile
   210 215 220
   Trp Pro Leu Leu Ala Lys Thr Pro Pro Gly Ala Gly Asp Glu Ile Gln
   225 230 235 240
   Leu Thr Asp Ala Ile Asp Met Leu Ile Glu Lys Glu Thr Val Glu Ala
   245 250 255
   Tyr His Met Lys Gly Lys Ser His Asp Cys Gly Asn Lys Leu Gly Tyr
   260 265 270
   Met Gln Ala Phe Val Glu Tyr Gly Ile Arg His Asn Thr Leu Gly Thr
   275 280 285
   Glu Phe Lys Ala Trp Leu Glu Glu Glu Met Gly Ile Lys Lys
   290 295 300
<210> 8
   <211> 546
   <212> PRT
   <213> Escherichia coli
   <400> 8
   Met Ala Ile His Asn Arg Ala Gly Gln Pro Ala Gln Gln Ser Asp Leu
   1               5                   10                  15
   Ile Asn Val Ala Gln Leu Thr Ala Gln Tyr Tyr Val Leu Lys Pro Glu
               20                  25                  30
   Ala Gly Asn Ala Glu His Ala Val Lys Phe Gly Thr Ser Gly His Arg
           35                  40                  45
   Gly Ser Ala Ala Arg His Ser Phe Asn Glu Pro His Ile Leu Ala Ile
       50                  55                  60
   Ala Gln Ala Ile Ala Glu Glu Arg Ala Lys Asn Gly Ile Thr Gly Pro
   65                  70                  75                  80
   Cys Tyr Val Gly Lys Asp Thr His Ala Leu Ser Glu Pro Ala Phe Ile
                   85                  90                  95
   Ser Val Leu Glu Val Leu Ala Ala Asn Gly Val Asp Val Ile Val Gln
               100                 105                 110
   Glu Asn Asn Gly Phe Thr Pro Thr Pro Ala Val Ser Asn Ala Ile Leu
           115                 120                 125
   Val His Asn Lys Lys Gly Gly Pro Leu Ala Asp Gly Ile Val Ile Thr
       130                 135                 140
   Pro Ser His Asn Pro Pro Glu Asp Gly Gly Ile Lys Tyr Asn Pro Pro
   145                 150                 155                 160
   Asn Gly Gly Pro Ala Asp Thr Asn Val Thr Lys Val Val Glu Asp Arg
                   165                 170                 175
   Ala Asn Ala Leu Leu Ala Asp Gly Leu Lys Gly Val Lys Arg Ile Ser
               180                 185                 190
   Leu Asp Glu Ala Met Ala Ser Gly His Val Lys Glu Gln Asp Leu Val
           195                 200                 205
   Gln Pro Phe Val Glu Gly Leu Ala Asp Ile Val Asp Met Ala Ala Ile
       210                 215                 220
   Gln Lys Ala Gly Leu Thr Leu Gly Val Asp Pro Leu Gly Gly Ser Gly
   225                 230                 235                 240
   Ile Glu Tyr Trp Lys Arg Ile Gly Glu Tyr Tyr Asn Leu Asn Leu Thr
                   245                 250                 255
   Ile Val Asn Asp Gln Val Asp Gln Thr Phe Arg Phe Met His Leu Asp
               260                 265                 270
   Lys Asp Gly Ala Ile Arg Met Asp Cys Ser Ser Glu Cys Ala Met Ala
           275                 280                 285
   Gly Leu Leu Ala Leu Arg Asp Lys Phe Asp Leu Ala Phe Ala Asn Asp
       290                 295                 300
   Pro Asp Tyr Asp Arg His Gly Ile Val Thr Pro Ala Gly Leu Met Asn
   305                 310                 315                 320
   Pro Asn His Tyr Leu Ala Val Ala Ile Asn Tyr Leu Phe Gln His Arg
                   325                 330                 335
   Pro Gln Trp Gly Lys Asp Val Ala Val Gly Lys Thr Leu Val Ser Ser
   340 345 350
   Ala Met Ile Asp Arg Val Val Asn Asp Leu Gly Arg Lys Leu Val Glu
   355 360 365
   Val Pro Val Gly Phe Lys Trp Phe Val Asp Gly Leu Phe Asp Gly Ser
   370 375 380
   Phe Gly Phe Gly Gly Glu Glu Ser Ala Gly Ala Ser Phe Leu Arg Phe
   385 390 395 400
   Asp Gly Thr Pro Trp Ser Thr Asp Lys Asp Gly Ile Ile Met Cys Leu
   405 410 415
   Leu Ala Ala Glu Ile Thr Ala Val Thr Gly Lys Asn Pro Gln Glu His
   420 425 430
   Tyr Asn Glu Leu Ala Lys Arg Phe Gly Ala Pro Ser Tyr Asn Arg Leu
   435 440 445
   Gln Ala Ala Ala Thr Ser Ala Gln Lys Ala Ala Leu Ser Lys Leu Ser
   450 455 460
   Pro Glu Met Val Ser Ala Ser Thr Leu Ala Gly Asp Pro Ile Thr Ala
   465 470 475 480
   Arg Leu Thr Ala Ala Pro Gly Asn Gly Ala Ser Ile Gly Gly Leu Lys
   485 490 495
   Val Met Thr Asp Asn Gly Trp Phe Ala Ala Arg Pro Ser Gly Thr Glu
   500 505 510
   Asp Ala Tyr Lys Ile Tyr Cys Glu Ser Phe Leu Gly Glu Glu His Arg
   515 520 525
   Lys Gln Ile Glu Lys Glu Ala Val Glu Ile Val Ser Glu Val Leu Lys
   530 535 540
   Asn Ala
   545
<210> 9
   <211> 558
   <212> PRT
   <213> Escherichia coli
   <400> 9
   Met Lys Leu Phe Lys Ser Ile Leu Leu Ile Ala Ala Cys His Ala Ala
   1               5                   10                  15
   Gln Ala Ser Ala Ala Ile Asp Ile Asn Ala Asp Pro Asn Leu Thr Gly
               20                  25                  30
   Ala Ala Pro Leu Thr Gly Ile Leu Asn Gly Gln Gln Ser Asp Thr Gln
           35                  40                  45
   Asn Met Ser Gly Phe Asp Asn Thr Pro Pro Pro Ser Pro Pro Val Val
       50                  55                  60
   Met Ser Arg Met Phe Gly Ala Gln Leu Phe Asn Gly Thr Ser Ala Asp
   65                  70                  75                  80
   Ser Gly Ala Thr Val Gly Phe Asn Pro Asp Tyr Ile Leu Asn Pro Gly
                   85                  90                  95
   Asp Ser Ile Gln Val Arg Leu Trp Gly Ala Phe Thr Phe Asp Gly Ala
               100                 105                 110
   Leu Gln Val Asp Pro Lys Gly Asn Ile Phe Leu Pro Asn Val Gly Pro
           115                 120                 125
   Val Lys Val Ala Gly Val Ser Asn Ser Gln Leu Asn Ala Leu Val Thr
       130                 135                 140
   Ser Lys Val Lys Glu Val Tyr Gln Ser Asn Val Asn Val Tyr Ala Ser
   145                 150                 155                 160
   Leu Leu Gln Ala Gln Pro Val Lys Val Tyr Val Thr Gly Phe Val Arg
                   165                 170                 175
   Asn Pro Gly Leu Tyr Gly Gly Val Thr Ser Asp Ser Leu Leu Asn Tyr
               180                 185                 190
   Leu Ile Lys Ala Gly Gly Val Asp Pro Glu Arg Gly Ser Tyr Val Asp
           195                 200                 205
   Ile Val Val Lys Arg Gly Asn Arg Val Arg Ser Asn Val Asn Leu Tyr
       210                 215                 220
   Asp Phe Leu Leu Asn Gly Lys Leu Gly Leu Ser Gln Phe Ala Asp Gly
   225                 230                 235                 240
   Asp Thr Ile Ile Val Gly Pro Arg Gln His Thr Phe Ser Val Gln Gly
                   245                 250                 255
   Asp Val Phe Asn Ser Tyr Asp Phe Glu Phe Arg Glu Ser Ser Ile Pro
               260                 265                 270
   Val Thr Glu Ala Leu Ser Trp Ala Arg Pro Lys Pro Gly Ala Thr His
           275                 280                 285
   Ile Thr Ile Met Arg Lys Gln Gly Leu Gln Lys Arg Ser Glu Tyr Tyr
       290                 295                 300
   Pro Ile Ser Ser Ala Pro Gly Arg Met Leu Gln Asn Gly Asp Thr Leu
   305                 310                 315                 320
   Ile Val Ser Thr Asp Arg Tyr Ala Gly Thr Ile Gln Val Arg Val Glu
                   325                 330                 335
   Gly Ala His Ser Gly Glu His Ala Met Val Leu Pro Tyr Gly Ser Thr
               340                 345                 350
   Met Arg Ala Val Leu Glu Lys Val Arg Pro Asn Ser Met Ser Gln Met
           355                 360                 365
   Asn Ala Val Gln Leu Tyr Arg Pro Ser Val Ala Gln Arg Gln Lys Glu
       370                 375                 380
   Met Leu Asn Leu Ser Leu Gln Lys Leu Glu Glu Ala Ser Leu Ser Ala
   385                 390                 395                 400
   Gln Ser Ser Thr Lys Glu Glu Ala Ser Leu Arg Met Gln Glu Ala Gln
                   405                 410                 415
   Leu Ile Ser Arg Phe Val Ala Lys Ala Arg Thr Val Val Pro Lys Gly
               420                 425                 430
   Glu Val Ile Leu Asn Glu Ser Asn Ile Asp Ser Val Leu Leu Glu Asp
           435                 440                 445
   Gly Asp Val Ile Asn Ile Pro Glu Lys Thr Ser Leu Val Met Val His
       450                 455                 460
   Gly Glu Val Leu Phe Pro Asn Ala Val Ser Trp Gln Lys Gly Met Thr
   465                 470                 475                 480
   Thr Glu Asp Tyr Ile Glu Lys Cys Gly Gly Leu Thr Gln Lys Ser Gly
                   485                 490                 495
   Asn Ala Arg Ile Ile Val Ile Arg Gln Asn Gly Ala Ala Val Asn Ala
               500                 505                 510
   Glu Asp Val Asp Ser Leu Lys Pro Gly Asp Glu Ile Met Val Leu Pro
           515                 520                 525
   Lys Tyr Glu Ser Lys Asn Ile Glu Val Thr Arg Gly Ile Ser Thr Ile
       530                 535                 540
   Leu Tyr Gln Leu Ala Val Gly Ala Lys Val Ile Leu Ser Leu
   545 550 555
<210> 10
   <211> 207
   <212> PRT
   <213> Escherichia coli
   <400> 10
   Met Ser Lys Lys Leu Ile Ile Phe Gly Ala Gly Gly Phe Ser Lys Ser
   1               5                   10                  15
   Ile Ile Asp Ser Leu Asn His Lys His Tyr Glu Leu Ile Gly Phe Ile
               20                  25                  30
   Asp Lys Tyr Lys Ser Gly Tyr His Gln Ser Tyr Pro Ile Leu Gly Asn
           35                  40                  45
   Asp Ile Ala Asp Ile Glu Asn Lys Asp Asn Tyr Tyr Tyr Phe Ile Gly
       50                  55                  60
   Ile Gly Lys Pro Ser Thr Arg Lys His Tyr Leu Asn Ile Ile Arg Lys
   65                  70                  75                  80
   His Asn Leu Arg Leu Ile Asn Ile Ile Asp Lys Thr Ala Ile Leu Ser
                   85                  90                  95
   Pro Asn Ile Ile Leu Gly Asp Gly Ile Phe Ile Gly Lys Met Cys Ile
               100                 105                 110
   Leu Asn Arg Asp Thr Arg Ile His Asp Ala Val Val Ile Asn Thr Arg
           115                 120                 125
   Ser Leu Ile Glu His Gly Asn Glu Ile Gly Cys Cys Ser Asn Ile Ser
       130                 135                 140
   Thr Asn Val Val Leu Asn Gly Asp Val Ser Val Gly Glu Glu Thr Phe
   145                 150                 155                 160
   Val Gly Ser Val Thr Val Val Asn Gly Gln Leu Lys Leu Gly Ser Lys
                   165                 170                 175
   Ser Ile Ile Gly Ser Gly Ser Val Val Ile Arg Asn Ile Pro Ser Asn
               180                 185                 190
   Val Val Val Ala Gly Thr Pro Thr Arg Leu Ile Arg Gly Asn Glu
           195                 200                 205
<210> 11
   <211> 191
   <212> PRT
   <213> Escherichia coli
   <400> 11
   Met Ala Lys Ser Val Pro Ala Ile Phe Leu Asp Arg Asp Gly Thr Ile
   1               5                   10                  15
   Asn Val Asp His Gly Tyr Val His Glu Ile Asp Asn Phe Glu Phe Ile
               20                  25                  30
   Asp Gly Val Ile Asp Ala Met Arg Glu Leu Lys Lys Met Gly Phe Ala
           35                  40                  45
   Leu Val Val Val Thr Asn Gln Ser Gly Ile Ala Arg Gly Lys Phe Thr
       50                  55                  60
   Glu Ala Gln Phe Glu Thr Leu Thr Glu Trp Met Asp Trp Ser Leu Ala
   65                  70                  75                  80
   Asp Arg Asp Val Asp Leu Asp Gly Ile Tyr Tyr Cys Pro His His Pro
                   85                  90                  95
   Gln Gly Ser Val Glu Glu Phe Arg Gln Val Cys Asp Cys Arg Lys Pro
               100                 105                 110
   His Pro Gly Met Leu Leu Ser Ala Arg Asp Tyr Leu His Ile Asp Met
           115                 120                 125
   Ala Ala Ser Tyr Met Val Gly Asp Lys Leu Glu Asp Met Gln Ala Ala
       130                 135                 140
   Val Ala Ala Asn Val Gly Thr Lys Val Leu Val Arg Thr Gly Lys Pro
   145                 150                 155                 160
   Ile Thr Pro Glu Ala Glu Asn Ala Ala Asp Trp Val Leu Asn Ser Leu
                   165                 170                 175
   Ala Asp Leu Pro Gln Ala Ile Lys Lys Gln Gln Lys Pro Ala Gln
               180                 185                 190
<210> 12
   <211> 310
   <212> PRT
   <213> Escherichia coli
   <400> 12
<210> 13
   <211> 477
   <212> PRT
   <213> Escherichia coli
   <400> 13
   Met Lys Val Thr Leu Pro Glu Phe Glu Arg Ala Gly Val Met Val Val
   1               5                   10                  15
   Gly Asp Val Met Leu Asp Arg Tyr Trp Tyr Gly Pro Thr Ser Arg Ile
               20                  25                  30
   Ser Pro Glu Ala Pro Val Pro Val Val Lys Val Asn Thr Ile Glu Glu
           35                  40                  45
   Arg Pro Gly Gly Ala Ala Asn Val Ala Met Asn Ile Ala Ser Leu Gly
       50                  55                  60
   Ala Asn Ala Arg Leu Val Gly Leu Thr Gly Ile Asp Asp Ala Ala Arg
   65                  70                  75                  80
   Ala Leu Ser Lys Ser Leu Ala Asp Val Asn Val Lys Cys Asp Phe Val
                   85                  90                  95
   Ser Val Pro Thr His Pro Thr Ile Thr Lys Leu Arg Val Leu Ser Arg
               100                 105                 110
   Asn Gln Gln Leu Ile Arg Leu Asp Phe Glu Glu Gly Phe Glu Gly Val
           115                 120                 125
   Asp Pro Gln Pro Leu His Glu Arg Ile Asn Gln Ala Leu Ser Ser Ile
       130                 135                 140
   Gly Ala Leu Val Leu Ser Asp Tyr Ala Lys Gly Ala Leu Ala Ser Val
   145                 150                 155                 160
   Gln Gln Met Ile Gln Leu Ala Arg Lys Ala Gly Val Pro Val Leu Ile
                   165                 170                 175
   Asp Pro Lys Gly Thr Asp Phe Glu Arg Tyr Arg Gly Ala Thr Leu Leu
               180                 185                 190
   Thr Pro Asn Leu Ser Glu Phe Glu Ala Val Val Gly Lys Cys Lys Thr
           195                 200                 205
   Glu Glu Glu Ile Val Glu Arg Gly Met Lys Leu Ile Ala Asp Tyr Glu
       210                 215                 220
   Leu Ser Ala Leu Leu Val Thr Arg Ser Glu Gln Gly Met Ser Leu Leu
   225 230 235 240
   Gln Pro Gly Lys Ala Pro Leu His Met Pro Thr Gln Ala Gln Glu Val
   245 250 255
   Tyr Asp Val Thr Gly Ala Gly Asp Thr Val Ile Gly Val Leu Ala Ala
   260 265 270
   Thr Leu Ala Ala Gly Asn Ser Leu Glu Glu Ala Cys Phe Phe Ala Asn
   275 280 285
   Ala Ala Ala Gly Val Val Val Gly Lys Leu Gly Thr Ser Thr Val Ser
   290 295 300
   Pro Ile Glu Leu Glu Asn Ala Val Arg Gly Arg Ala Asp Thr Gly Phe
   305 310 315 320
   Gly Val Met Thr Glu Glu Glu Leu Lys Leu Ala Val Ala Ala Ala Arg
   325 330 335
   Lys Arg Gly Glu Lys Val Val Met Thr Asn Gly Val Phe Asp Ile Leu
   340 345 350
   His Ala Gly His Val Ser Tyr Leu Ala Asn Ala Arg Lys Leu Gly Asp
   355 360 365
   Arg Leu Ile Val Ala Val Asn Ser Asp Ala Ser Thr Lys Arg Leu Lys
   370 375 380
   Gly Asp Ser Arg Pro Val Asn Pro Leu Glu Gln Arg Met Ile Val Leu
   385 390 395 400
   Gly Ala Leu Glu Ala Val Asp Trp Val Val Ser Phe Glu Glu Asp Thr
   405 410 415
   Pro Gln Arg Leu Ile Ala Gly Ile Leu Pro Asp Leu Leu Val Lys Gly
   420 425 430
   Gly Asp Tyr Lys Pro Glu Glu Ile Ala Gly Ser Lys Glu Val Trp Ala
   435 440 445
   Asn Gly Gly Glu Val Leu Val Leu Asn Phe Glu Asp Gly Cys Ser Thr
   450 455 460
   Thr Asn Ile Ile Lys Lys Ile Gln Gln Asp Lys Lys Gly
   465 470 475
<210> 14
   <211> 420
   <212> PRT
   <213> Escherichia coli
   <400> 14
   Met Leu Lys Arg Leu Gly Lys Val Phe Gly Pro Leu Val Cys Ala Leu
   1               5                   10                  15
   Leu Leu Leu Val Gly Leu Tyr Leu Val Phe Pro Val Ser Gln Pro His
               20                  25                  30
   His Leu Gly Lys Glu Lys Asn Ser Ala Val Ala Leu Thr Lys Ala Gly
           35                  40                  45
   Phe Lys Ser Arg Val Gln Lys Val Arg Ala Phe Ser Asp Pro Lys Ala
       50                  55                  60
   Asn Phe Val Pro Phe Phe Gly Ser Ser Glu Trp Leu Arg Phe Asp Ala
   65                  70                  75                  80
   Met His Pro Ser Val Leu Ala Glu Ala Tyr Lys Arg Pro Tyr Ile Pro
                   85                  90                  95
   Tyr Leu Leu Gly Gln Lys Gly Ala Ala Ser Leu Thr Gln Tyr Tyr Gly
               100                 105                 110
   Ile Gln Gln Ile Lys Gly Gln Ile Lys Asn Lys Lys Ala Ile Tyr Val
           115                 120                 125
   Ile Ser Pro Gln Trp Phe Val Arg Lys Gly Ala Asn Lys Gly Ala Phe
       130                 135                 140
   Gln Asn Tyr Phe Ser Asn Asp Gln Thr Ile Arg Phe Leu Gln Asn Gln
   145                 150                 155                 160
   Thr Gly Thr Thr Tyr Asp Arg Tyr Ala Ala Arg Arg Leu Leu Lys Leu
                   165                 170                 175
   Tyr Pro Glu Ala Ser Met Ser Asp Leu Ile Glu Lys Val Ala Asp Gly
               180                 185                 190
   Gln Lys Leu Ser Asn Lys Asp Lys Gln Arg Leu Lys Phe Asn Asp Trp
           195                 200                 205
   Val Phe Glu Lys Thr Asp Ala Ile Phe Ser Tyr Leu Pro Leu Gly Lys
       210                 215                 220
   Thr Tyr Asn Gln Val Ile Met Pro His Val Gly Lys Leu Pro Lys Ala
   225                 230                 235                 240
   Phe Ser Tyr Asn His Leu Ser Arg Ile Ala Ser Gln Asp Ala Lys Val
                   245                 250                 255
   Ala Thr Arg Ser Asn Gln Phe Gly Ile Asp Asp Arg Phe Tyr Gln Thr
               260                 265                 270
   Arg Ile Lys Lys His Leu Lys Lys Leu Lys Gly Ser Gln Arg His Phe
           275                 280                 285
   Asn Tyr Thr Lys Ser Pro Glu Phe Asn Asp Leu Gln Leu Val Leu Asn
       290                 295                 300
   Glu Phe Ser Lys Gln Asn Thr Asp Val Leu Phe Val Ile Pro Pro Val
   305                 310                 315                 320
   Asn Lys Lys Trp Thr Asp Tyr Thr Gly Leu Asp Gln Lys Met Tyr Gln
                   325                 330                 335
   Lys Ser Val Glu Lys Ile Lys His Gln Leu Gln Ser Gln Gly Phe Asn
               340                 345                 350
   His Ile Ser Asp Leu Ser Arg Asp Gly Gly Lys Pro Tyr Phe Met Gln
           355                 360                 365
   Asp Thr Ile His Leu Gly Trp Asn Gly Trp Leu Glu Leu Asp Lys His
       370                 375                 380
   Ile Asn Pro Phe Leu Thr Glu Glu Asn Ser Lys Pro Asn Tyr His Ile
   385                 390                 395                 400
   Asn Asn Lys Phe Leu Lys Arg Ser Trp Ala Lys Tyr Thr Gly Arg Pro
                   405                 410                 415
   Ser Asp Tyr Lys
               420
<210> 15
   <211> 511
   <212> PRT
   <213> Escherichia coli
   <400> 15
<210> 16
   <211> 919
   <212> DNA
   <213> Escherichia coli
   <400> 16
   gcccgcactc actgatgccc agcaggcaat cccagggatt aagtttgact gggtggtgga 60
   agaagggttc gcacagattc cttcctggca cgctgccgtt gagcgagtta ttcctgtggc 120
   aatacgtcgc tggcgtaaag cctggttctc ggcccccata aaagctgaac gcaaagcgtt 180
   tcgtgaagcg ctacaagcag agaactatga cgcagttatc gacgctcagg ggctggtaaa 240
   aagcgcggca ctggtgacac gtctggcgca tggcgtaaag catggattgg actggcaaac 300
   cgctcgcgaa cctttagcca gcctgtttta caattgtaag catcatattg caaaacagca 360
   gcacgccgta gaacgcaccc gcgaactgtt tgccaaaagt ttgggctata gcaaaccgca 420
   aacccagggc gattatgcta tcgcacagca ttttctgacg aacctgccta cagatgctgg 480
   cgaatatgcc gtatttcttc atgcgacgac ccgtgatgat aaacactggc cggaagaaca 540
   ctggcgagaa ttgattggtt tactggctga ttcaggaata cggattaaac ttccgtgggg 600
   cgcgccgcat gaggaagaac gggcgaaacg actggcggaa ggatttgctt atgttgaagt 660
   attgccgaag atgagtctgg aaggcgttgc ccgcgtgctg gccggggcta aatttgtagt 720
   gtcggtggat acggggttaa gccatttaac ggcggcactg gatagaccca atatcacggt 780
   ttatggacca accgatccgg gattaattgg tgggtatggg aagaatcaga tggtttgtag 840
   ggctccgggg aatgagttgt ctcaattgac agcaaatgct gttaagcggt tcattgaaga 900
   aaacgctgcc atgatttaa 919
<210> 17
   <211> 1023
   <212> DNA
   <213> Escherichia coli
   <400> 17
   atgcgttttc atggggatat gttattaact actcccgtca ttagttcgct gaaaaaaaat 60
   taccctgacg caaaaatcga tgtgctgctt tatcaggaca ccatcccgat cctgtctgaa 120
   aatccagaga ttaacgcgct ctacggcata aaaaataaaa aagcaaaagc ctcagaaaaa 180
   attgccaact tttttcatct catcaaggta ttacgtgcca ataagtatga ccttatcgtc 240
   aatctcaccg atcaatggat ggttgctata ctggttcgct tattaaatgc ccgtgtgaaa 300
   atttcccagg attatcatca tcggcagtct gctttttggc gtaaaagttt cacccatttg 360
   gtgccgttgc agggtggaaa tgtggtggaa agtaacttat ccgtgctgac cccattggga 420
   gttgattcgt tggtgaagca gacaaccatg agttacccgc ctgcaagctg gaaacgtatg 480
   cgtcgcgaac ttgatcacgc tggtgttgga caaaattatg tggttatcca acctacggcg 540
   cggcaaatct tcaaatgctg ggacaacgcc aagttttccg ctgtgattga tgccttacat 600
   gctcgtggtt atgaagttgt tctgacgtcc ggcccagata aagacgatct ggcctgcgtc 660
   aatgaaattg cgcagggatg ccagacgcca ccagtaacgg cgctggctgg aaaggtgacc 720
   ttcccggaac ttggtgcgtt aatcgatcat gcgcagctgt ttattggcgt tgattccgca 780
   ccggcgcata ttgccgctgc agttaatacg ccgctgatat cgctgtttgg tgcgacagac 840
   catattttct ggcgtccctg gtcaaataac atgattcaat tctgggcggg agattaccgg 900
   gaaatgccaa cgcgcgatca gcgtgaccga aatgagatgt atctttcggt tattccggcg 960
   gcagatgtca ttgctgctgt cgataaatta ctgccctcct ccacgacagg tacgtcgtta 1020
   tga 1023
<210> 18
   <211> 798
   <212> DNA
   <213> Escherichia coli
   <400> 18
   atggttgaac ttaaagagcc gtttgccacg ttatggcgcg gcaaagatcc ttttgaggaa     60
   gttaaaacct tgcagggtga ggtatttcgt gaactggaaa ctcgccgtac tctgcgcttt    120
   gaaatggcgg gcaaaagcta ttttctcaaa tggcatcgcg gcacgaccct gaaagagata    180
   atcaaaaatt tactctcatt gcggatgcca gtattaggcg ctgaccgcga atggaatgcg    240
   attcatcgac tgcgggatgt cggcgttgat actatgtatg gggtggcatt tggcgaaaaa     300
   ggcatgaatc cgctgaccag aacttcattt attattaccg aagatctgac accaaccata    360
   agtctggaag attactgtgc tgactgggcg actaaccctc cagatgttcg cgtaaagcgt    420
   atgcttatta agcgtgtcgc gacgatggtg cgcgatatgc atgctgcggg cattaaccac    480
   cgtgactgtt atatctgtca tttcctgctg cacttgcctt tttccggtaa ggaagaggag    540
   ttaaaaattt cggtaattga cctgcaccgg gcgcagcttc gcacgcgcgt tccacgtcgt    600
   tggcgggata aagatcttat tgggctttat ttttcttcga tgaatatcgg cctgactcag    660
   cgggatatct ggcggtttat gaaagtgtat tttgccgccc cgcttaaaga cattctcaag    720
   caggaacaag gactgctgtc gcaagcagaa gcaaaagcca caaaaatcag ggaaagaacg    780
   attcgaaaat cgttgtaa                                                  798
<210> 19
   <211> 1125
   <212> DNA
   <213> Escherichia coli
   <400> 19
   atgatcgttg ctttttgttt atataaatat tttccctttg gcggtttgca gcgcgatttt      60
   atgcgtattg ctcagacagt cgccgcccga ggtcatcatg ttcgggttta tacccagtcg     120
   tgggaaggcg aatgccctga tgtatttgaa ctgatcaaag tgccggttaa atcgcatacc     180
   aatcacgggc gcaatgcgga gtattttgcc tgggtgcaaa aacatttacg cgaacatccc     240
   gtcgataaag tcgttggatt caacaaaatg ccggggctgg acgtttatta tgccgctgat     300
   gtttgttatg ccgagaaagt agcgcaggaa aaaggctttt tctatcgcct gacgtcacgt     360
   tatcgccatt atgccgcctt tgagcgggca accttcgaac agggcaagcc gacacagctg     420
   ctgatgctga cagataagca aatcgccgat ttccagaaac attatcagac tgaagcggag     480
   cgttttcata ttctgccacc ggggatttat cctgatcgta aatatagcca gcagccagcc     540
   aatagccgtg aaatcttccg taagaagaat ggaataaccg aacaacaata tttattgttg     600
   caggtcggtt cagacttcac gcgtaaaggt gtcgatcgtt ccattgaagc acttgcttcg     660
   ttaccggatt cgctgcgcca caacacattg ctatatgttg ttgggcagga taaaccgcga     720
   aaatttgagg cactggcaga aaaacgcggc gtgcgcagta atgttcactt cttctcgggg     780
   cgcaacgatg tctcggaatt aatggcggcg gcggatttat tactgcatcc tgcctaccag     840
   gaagcggcgg gaattgtgct gctggaagcg attactgcag gattaccggt actaacaact     900
   gccgtttgtg gctatgcgca ttatattgtc gacgctaatt gcggcgaggc tattgctgag     960
   ccattccgcc aggaaacatt gaatgagatt ttacgcaaag cgttaacgca atcttcattg    1020
   cgccaggctt gggcggaaaa tgcgcgacat tatgctgata cacaagattt atacagtctg    1080
   ccagagaaag cggcggatat cataacgggt ggtctggatg gttga                    1125
<210> 20
   <211> 1047
   <212> DNA
   <213> Escherichia coli
   <400> 20
   atgaaaatac tggtgatcgg cccgtcttgg gttggcgaca tgatgatgtc gcaaagtctc     60
   tatcgcacgc tccaggcgcg ctatccccag gcgataatcg atgtgatggc accggcatgg    120
   tgccgtccat tattatcgcg gatgccggaa gttaacgaag ctattcctat gcctctcggt    180
   cacggagcgc tggaaatcgg cgaacgccgc aaactgggtc atagcctgcg tgaaaagcgc    240
   tacgaccgcg cctacgtctt acccaactcc ttcaaatctg cattagtgcc tttcttcgcg    300
   ggtattcctc atcgcaccgg ctggcgcggc gagatgcgct acggtttact caacgatgta    360
   cgcgtgctcg ataaagaagc ctggccgcta atggtggaac gctatatagc gctggcctat    420
   gacaaaggca ttatgcgcac agcacaagat ctgccgcagc cattgttatg gccgcagttg    480
   caggtgagcg aaggtgaaaa atcatatacc tgtaatcaat tttcgctttc atcagaacgt    540
   ccgatgattg gtttttgccc gggtgcggag tttggtccgg caaaacgctg gccacactac    600
   cactatgcgg agctggcaaa gcagctgatt gatgaaggtt atcaggtggt tctgtttggc    660
   tcggcgaaag atcatgaagc gggcaatgag attcttgccg ctttgaatac cgagcagcag    720
   gcatggtgtc ggaacctggc gggggaaaca cagcttgatc aagcggttat cctgattgca    780
   gcctgtaaag ccattgtcac taacgattct ggcctgatgc atgttgcggc ggcgctcaat    840
   cgtccgctgg ttgccctgta tggtccgagt agcccggact tcacaccgcc gctatcccat    900
   aaagcgcgcg tgatccgttt gattaccggc tatcacaaag tgcgtaaagg tgacgctgcg    960
   gagggttatc accagagctt aatcgacatt actccccagc gcgtactgga agaactcaac   1020
   gcgctattgt tacaagagga agcctga                                       1047
<210> 21
   <211> 1017
   <212> DNA
   <213> Escherichia coli
   <400> 21
   atgagtgccc actattttaa tccacaagag atgatcaata agacaatcat cttcgatgaa     60
   aggccagcgg cgtcagtggc atcatcattc catgttgctt atggcattga taaaaacttt    120
   ctttttggtt gtggtgtttc aatcacgtca gttttgttac ataacaacga cgtgagtttt    180
   gttttccacg tttttattga tgatatccct gaagccgata tccagcgttt agcccaattg    240
   gcgaaaagct atcgtacctg tatccagatc catctagtaa attgtgaacg gcttaaggca    300
   ttaccgacga ccaaaaattg gtctattgcc atgtatttcc gttttgtaat tgcagattac    360
   tttattgatc aacaagataa gatcctttac ctggatgctg atatcgcctg tcagggaaac    420
   ttaaagccgc tgataacaat ggatcttgcc aataacgttg ctgctgttgt tactgaacgc    480
   gatgctaact ggtggtcgtt acggggtcaa agtctgcagt gtaatgaact tgaaaagggt    540
   tactttaatt caggtgtcct gttaattaat acactagcgt gggcgcagga gtccgtttct    600
   gctaaagcga tgtcgatgct tgctgataaa gccatcgttt cccgtttaac ctatatggat    660
   caagatatcc ttaatcttat cctgttaggg aaagttaaat tcattgatgc taaatacaat    720
   acgcaattta gtttaaatta tgaattaaaa aaatcatttg tttgtccaat taatgatgaa    780
   accgtattaa ttcattatgt cggcccgaca aaaccctggc attactgggc cggttatcca    840
   agtgcgcaac cttttatcaa agccaaagaa gcatcgccct ggaaaaatga accgttaatg    900
   cggccagtta actcaaacta tgctcgttat tgcgccaagc ataattttaa acaaaacaaa    960
   ccaattaacg ggataatgaa ttatatttat tatttttatt taaagataat aaaatga      1017
<210> 22
   <211> 909
   <212> DNA
   <213> Escherichia coli
   <400> 22
   atggctgcca ttaatacgaa agtcaaaaaa gccgttatcc ccgttgcggg attaggaacc 60
   aggatgttgc cggcgacgaa agccatcccg aaagagatgc tgccacttgt cgataagcca 120
   ttaattcaat acgtcgtgaa tgaatgtatt gcggctggca ttactgaaat tgtgctggtt 180
   acacactcat ctaaaaactc tattgaaaac cactttgata ccagttttga actggaagca 240
   atgctggaaa aacgtgtaaa acgtcaactg cttgatgaag tgcagtctat ttgtccaccg 300
   cacgtgacta ttatgcaagt tcgtcagggt ctggcgaaag gcctgggaca cgcggtattg 360
   tgtgctcacc cggtagtggg tgatgaaccg gtagctgtta ttttgcctga tgttattctg 420
   gatgaatatg aatccgattt gtcacaggat aacctggcag agatgatccg ccgctttgat 480
   gaaacgggtc atagccagat catggttgaa ccggttgctg atgtgaccgc atatggcgtt 540
   gtggattgca aaggcgttga attagcgccg ggtgaaagcg taccgatggt tggtgtggta 600
   gaaaaaccga aagcggatgt tgcgccgtct aatctcgcta ttgtgggtcg ttacgtactt 660
   agcgcggata tttggccgtt gctggcaaaa acccctccgg gagctggtga tgaaattcag 720
   ctcaccgacg caattgatat gctgatcgaa aaagaaacgg tggaagccta tcatatgaaa 780
   gggaagagcc atgactgcgg taataaatta ggttacatgc aggccttcgt tgaatacggt 840
   attcgtcata acacccttgg cacggaattt aaagcctggc ttgaagaaga gatgggcatt 900
   aagaagtaa 909
<210> 23
   <211> 1641
   <212> DNA
   <213> Escherichia coli
   <400> 23
   atggcaatcc acaatcgtgc aggccaacct gcacaacaga gtgatttgat taacgtcgcc     60
   caactgacgg cgcaatatta tgtactgaaa ccagaagcag ggaatgcgga gcacgcggtg    120
   aaattcggta cttccggtca ccgtggcagt gcagcgcgcc acagctttaa cgagccgcac    180
   attctggcga tcgctcaggc aattgctgaa gaacgtgcga aaaacggcat cactggccct    240
   tgctatgtgg gtaaagatac tcacgccctg tccgaacctg cattcatttc cgttctggaa    300
   gtgctggcag cgaacggcgt tgatgtcatt gtgcaggaaa acaatggctt caccccgacg    360
   cctgccgttt ccaatgccat cctggttcac aataaaaaag gtggcccgct ggcagacggt    420
   atcgtgatta caccgtccca taacccgccg gaagatggtg gaatcaaata caatccgcca    480
   aatggtggcc cggctgatac caacgtcact aaagtggtgg aagacagggc caacgcactg    540
   ctggccgatg gcctgaaagg cgtgaagcgt atctccctcg acgaagcgat ggcatccggt    600
   catgtgaaag agcaggatct ggtgcagccg ttcgtggaag gtctggccga tatcgttgat    660
   atggccgcga ttcagaaagc gggcctgacg ctgggcgttg atccgctggg cggttccggt    720
   atcgaatact ggaagcgtat tggcgagtat tacaacctca acctgactat cgttaacgat    780
   caggtcgatc aaaccttccg ctttatgcac cttgataaag acggcgcgat ccgtatggac    840
   tgctcctccg agtgtgcgat ggcgggcctg ctggcactgc gtgataagtt cgatctggcg    900
   tttgctaacg acccggatta tgaccgtcac ggtatcgtca ctccggcagg tttgatgaat    960
   ccgaaccact acctggcggt ggcaatcaat tacctgttcc agcatcgtcc gcagtggggc   1020
   aaagatgttg ccgtcggtaa aacgctggtt tcatctgcga tgatcgaccg tgtggtcaac   1080
   gacttgggcc gtaaactggt agaagtcccg gtaggtttca aatggtttgt cgatggtctg   1140
   ttcgacggca gcttcggctt tggcggcgaa gagagtgcag gggcttcctt cctgcgtttc   1200
   gacggcacgc cgtggtccac cgacaaagac ggcatcatca tgtgtctgct ggcggcggaa   1260
   atcaccgctg tcaccggtaa gaacccgcag gaacactaca acgaactggc aaaacgcttt   1320
   ggtgcgccga gctacaaccg tttgcaggca gctgcgactt ccgcacaaaa agcggcgctg   1380
   tctaagctgt ctccggaaat ggtgagcgcc agcaccctgg caggtgaccc gatcaccgcg   1440
   cgcctgactg ctgctccggg caacggtgct tctattggcg gtctgaaagt gatgactgac   1500
   aacggctggt tcgccgcgcg tccgtcaggc acggaagacg catataagat ctactgcgaa   1560
   agcttcctcg gtgaagaaca tcgcaagcag attgagaaag aagcggttga gattgttagc   1620
   gaagttctga aaaacgcgta a                                             1641
<210> 24
   <211> 1677
   <212> DNA
   <213> Escherichia coli
   <400> 24
   atgaaattat ttaaatcaat tttactgatt gccgcctgtc acgcggcgca ggccagcgcg      60
   gccattgata ttaacgctga cccaaacctt acaggagccg cgccgcttac cggtattctg     120
   aacgggcaac agtcggatac gcaaaacatg agcggcttcg acaatacccc gccgccctca     180
   ccgccggtgg taatgagccg tatgtttggt gctcaacttt tcaacggcac cagcgcggat     240
   agcggtgcga cggtaggatt caaccctgac tatattctga atccgggtga tagcattcag     300
   gttcgcttgt ggggtgcgtt cacctttgat ggtgcgttac aggttgatcc caaaggtaat     360
   attttcctgc cgaacgttgg tccggtgaaa gttgctggcg tcagtaatag tcagctaaat     420
   gccctggtca catccaaagt gaaggaagta taccagtcca acgtcaacgt ctacgcctcc     480
   ttattacagg cgcagccagt aaaagtgtac gtgaccggat ttgtgcgtaa tcctggtctg     540
   tatggcggtg tgacgtctga ttcgttactc aattatctga tcaaggctgg cggcgttgat     600
   ccagagcgcg gaagttacgt tgatattgtg gtcaagcgcg gtaaccgcgt gcgctccaac     660
   gtcaacctgt acgacttcct gctgaacggc aaactggggc tttcgcagtt cgccgatggt     720
   gacaccatca tcgtcgggcc gcgtcagcat actttcagcg ttcagggcga tgtctttaac     780
   agctacgact ttgagttccg cgaaagcagc attcccgtaa cggaagcgtt gagctgggcg     840
   cgccctaagc ctggcgcgac tcacattacg attatgcgta aacaggggct gcaaaaacgc     900
   agcgaatact atccgatcag ttctgcgcca ggccgtatgt tgcaaaatgg cgatacctta     960
   atcgtgagca ctgaccgcta tgccggcacc attcaggtgc gggttgaagg cgcacactcc    1020
   ggtgaacatg ccatggtact gccttatggt tccactatgc gtgcggttct ggaaaaagtc    1080
   cgcccgaaca gcatgtcgca gatgaacgcg gttcagcttt atcgcccatc agtagctcag    1140
   cgtcagaaag agatgctgaa tctctcgctg caaaaactgg aggaagcatc actttctgcc    1200
   cagtcctcca ccaaagaaga agccagcctg cgaatgcagg aagcgcaact gatcagccgc    1260
   tttgtggcga aagcacgcac cgtagttccg aaaggtgaag tgatcctcaa cgaatccaat    1320
   attgattctg ttctgcttga agatggcgac gtcatcaata ttccggagaa aacatcgctg    1380
   gttatggttc atggcgaagt gctgttcccg aacgcggtga gctggcagaa gggtatgacc    1440
   accgaggatt acatcgagaa atgtggtggc ctgacgcaga aatcgggtaa cgccagaatt    1500
   atcgtcattc gtcagaacgg tgctgccgtc aacgcagaag atgtggattc actcaaaccg    1560
   ggcgatgaga ttatggttct gccgaaatat gaatcgaaaa acattgaagt tacccgtggt    1620
   atttccacca tcctctatca gctggcggtg ggtgcaaaag tgattctgtc tttgtaa       1677
<210> 25
   <211> 624
   <212> DNA
   <213> Escherichia coli
   <400> 25
   atgagtaaaa agttaataat atttggtgcg ggtggttttt caaaatctat aattgacagc 60
   ttaaatcata aacattacga gttaatagga tttatcgata aatataaaag tggttatcat 120
   caatcatatc caatattagg taatgatatt gcagacatcg agaataagga taattattat 180
   tattttattg ggataggcaa accatcaact aggaagcact atttaaacat cataagaaaa 240
   cataatctac gcttaattaa cattatagat aaaactgcta ttctatcacc aaatattata 300
   ctgggtgatg gaatttttat tggtaaaatg tgtatactta accgtgatac tagaatacat 360
   gatgccgttg taataaatac taggagttta attgaacatg gtaatgaaat aggctgctgt 420
   agcaatatct ctactaatgt tgtacttaat ggtgatgttt ctgttggaga agaaactttt 480
   gttggtagcg tgactgttgt aaatggccag ttgaagctag gctcaaagag tattattggt 540
   tctgggtcgg ttgtaattag aaatatacca agtaatgttg tagttgctgg gactccaaca 600
   agattaatta gggggaatga atga 624
<210> 26
   <211> 576
   <212> DNA
   <213> Escherichia coli
   <400> 26
   gtggcgaaga gcgtacccgc aatttttctt gaccgtgatg gcaccattaa tgtcgatcac 60
   ggctatgtcc atgagatcga caactttgaa tttatcgacg gtgttattga cgccatgcgc 120
   gagctaaaaa aaatgggctt tgcgctggtg gtagtcacca accagtctgg cattgctcgc 180
   ggtaaattta ccgaagcaca gtttgaaacg ctgaccgagt ggatggactg gtcgctggcg 240
   gaccgagatg tcgatctgga tggtatctat tattgcccgc atcatccgca gggtagtgtt 300
   gaagagtttc gccaggtctg cgattgccgc aaaccacatc cggggatgct tttgtcagca 360
   cgcgattatt tgcatattga tatggccgct tcttatatgg tgggcgataa attagaagat 420
   atgcaggcag cggttgcggc gaacgtggga acaaaagtgc tggtgcgtac gggtaaacct 480
   attacacctg aagcagaaaa cgcggcagat tgggtgttaa atagcctggc agacctgccg 540
   caagcgataa aaaagcagca aaaaccggcg caatga 576
<210> 27
   <211> 933
   <212> DNA
   <213> Escherichia coli
   <400> 27
   atgatcatcg ttaccggcgg cgcgggcttt atcggcagca acatcgttaa agccctgaat      60
   gataaaggca tcaccgatat tctggtggtg gacaacctga aagacggcac caagtttgtg     120
   aacctggtgg atctgaatat cgcagactat atggataagg aagacttcct gatccagatt     180
   atggctggcg aagagttcgg cgatgtcgaa gcgattttcc acgaaggcgc gtgctcttcc     240
   accaccgagt gggacggcaa gtatatgatg gataacaact atcaatactc caaggagctg     300
   ctgcactact gcctggagcg tgaaatcccg ttcctgtacg cttcttccgc agccacctac     360
   ggcggacgca cctccgactt tattgaatcc cgcgagtacg aaaaaccgtt gaacgtctac     420
   ggttactcaa aattcctgtt tgatgaatat gttcgccaaa tcctgccgga agcgaactcg     480
   cagattgttg gcttccgcta tttcaacgtt tatggaccgc gtgaaggcca taaaggcagc     540
   atggcgagcg tcgctttcca tctcaacacc cagcttaata acggtgaatc accgaagctg     600
   ttcgaaggta gcgagaactt taaacgcgac ttcgtctatg ttggcgatgt ggcagatgta     660
   aacctgtggt tcctggaaaa tggcgtttcc ggcatcttca acctcggcac cggtcgtgcg     720
   gaatccttcc aggctgtagc tgatgctacg ctggcttatc acaagaaagg ccagatcgaa     780
   tacattccgt tcccggataa gctgaaaggc cgctaccagg cgttcactca ggcagatctg     840
   acaaatctgc gcgcggcggg ttacgacaaa ccgttcaaaa ccgttgctga aggtgtaacg     900
   gaatacatgg cctggctgaa tcgcgacgca taa                                  933
<210> 28
   <211> 1434
   <212> DNA
   <213> Escherichia coli
   <400> 28
<210> 29
   <211> 1263
   <212> DNA
   <213> Escherichia coli
   <400> 29
   atgcttaaac gtctaggtaa agtatttgga cctctagttt gtgctttact attgttggta      60
   ggattatatc ttgtttttcc tgtttctcag cctcatcatt taggtaagga aaaaaacagt     120
   gcagtagcgt tgacaaaggc aggttttaaa agcagagttc aaaaagttag agctttcagt     180
   gatcctaaag ccaattttgt ccctttcttt ggttcaagtg agtggttaag atttgatgca     240
   atgcatccat cagttttagc agaggcttac aaaaggcctt atatcccata tcttttaggt     300
   caaaaagggg cggcttctct gacacaatac tatggcattc aacagattaa aggacaaatc     360
   aaaaataaaa aagctatcta tgttatttct ccgcaatggt ttgttcgcaa gggagccaac     420
   aaaggtgctt ttcaaaacta tttcagcaac gatcaaacca ttcgattttt gcaaaatcaa     480
   acagggacaa cctacgatag gtatgctgct cgtcgattgt taaaattata tcctgaagct     540
   tctatgtcag atttgataga aaaagttgca gatggccaaa aactatcaaa taaagacaaa     600
   caaagactaa agtttaatga ttgggtattt gagaagacag atgctatttt tagctatcta     660
   ccactaggaa aaacttataa tcaggtaata atgcctcatg ttggtaaatt accgaaagca     720
   ttctcatata atcatttatc gcgtattgca tcacaagatg ctaaagtagc aacgagatca     780
   aatcaatttg gtattgatga tcgcttttac caaacaagaa ttaaaaagca cttaaaaaaa     840
   ttgaaagggt cacaacgaca tttcaattat actaagtcac cagaatttaa tgatttacag     900
   ttggttctta atgaattctc aaaacaaaat acagatgtcc tttttgtcat accaccagta     960
   aataaaaagt ggacagacta cacagggctt gatcaaaaaa tgtatcaaaa atctgtagaa    1020
   aaaataaaac accaacttca aagtcaaggt ttcaatcata tctctgacct ttctcgagat    1080
   ggaggtaagc catactttat gcaagataca atccatttag gttggaatgg ttggttagag    1140
   ctagataagc atatcaatcc atttttaaca gaggaaaaca gcaagccaaa ttatcacatt    1200
   aataataaat ttttgaagag atcttgggca aaatatacag gacgtccaag tgattacaag    1260
   taa                                                                  1263
<210> 30
   <211> 1536
   <212> DNA
   <213> Escherichia coli
   <400> 30
   atgatacatg atatgattaa aacaattgag cattttgctg agacacaagc tgattttcca      60
   gtgtatgata ttttagggga agtccatact tatggacaac ttaaagtaga ctctgactct     120
   ctagctgctc atattgatag cctaggcctt gttgaaaaat cacctgtctt agtattcggt     180
   ggtcaagaat atgaaatgtt ggcgacattt gttgctttaa caaagtcagg gcatgcttat     240
   ataccggttg accaacactc tgctttggat agaatacagg ctattatgac agttgctcaa     300
   ccaagcctta tcatttcaat tggtgaattt cctcttgaag ttgataatgt cccaatccta     360
   gacgtttctc aagtttcagc tatttttgaa gaaaagactc cttatgaggt aacacattct     420
   gttaaaggtg atgataatta ctatattatt ttcacttcag ggactactgg tttaccaaaa     480
   ggtgtgcaaa tttcacatga caatttattg agctttacaa attggatgat ttctgatgat     540
   gagttttcag ttcctgaaag accgcaaatg ttggctcaac cgccatattc atttgactta     600
   tcagttatgt attgggcacc aacactagct atgggaggca ccctgtttgc cctaccaaaa     660
   acagtagtta atgatttcaa aaaactattc gctaccatta atgaattgcc aatacaggtt     720
   tggacttcga caccatcatt tgctgatatg gcgctactat ctaacgattt caattcagag     780
   accttgccac agttaacaca tttttatttt gatggggaag agttaactgt caagactgca     840
   caaaaacttc gtcaacgttt tccaaaagct cgtatcgtta atgcatatgg gccaacagaa     900
   gcaacagttg ctctatccgc agtagcaatt actgatgaaa tgttagaaac atgcaaacgc     960
   cttccaattg gttacactaa agatgactca ccaacgtatg tgattgatga agaaggtcat    1020
   aaattaccaa acggagagca aggtgaaatc attattgctg gaccagcagt atcaaaaggc    1080
   tatcttaaca atccagaaaa gacagctgag gcatttttcc aattcgaagg tctacctgct    1140
   taccataccg gtgacttagg aagtatgacc gatgaaggtc ttctgcttta cggtgggcgt    1200
   atggatttcc aaattaaatt taacggctat cgtattgaat tagaagatgt ttctcaaaac    1260
   ttaaacaaat cgcagtatgt aaaatcagca gtagcagtgc cacgttataa caaggatcat    1320
   aaagttcaaa acttattagc ctatattgtc ttaaaagaag gtgtaagaga tgattttgaa    1380
   cgtgatttgg atttgacaaa agcaattaag gaagacttaa aggacattat gatggattac    1440
   atgatgccat ctaaatttat ctatcgagag gatttacctt tgacaccaaa tgggaaaatt    1500
   gatatcaaag gtcttatgag cgaggtaaac aagtga                               1536
<210> 31
   <211> 60
   <212> DNA
   <213> Escherichia coli
   <400> 31
   tcgtgcaggc caacctgcac aacagagtga tttgattaac gtgtaggctg gagctgcttc 60
<210> 32
   <211> 60
   <212> DNA
   <213> Escherichia coli
   <400> 32
   cagggtgctg gcgctcacca tttccggaga cagcttagac acatatgaat atcctcctta 60